# EUROPÄISCHE PATENTSCHRIFT

(11) **EP 1 506 169 B1**
(45) Veröffentlichungstag und Bekanntmachung des Hinweises auf die Patenterteilung: **23.12.2009**
(21) Anmeldenummer: 03730050.6
(22) Anmeldetag: 15.05.2003
(51) Int. Cl.: C07C 307/02, C07C 303/34

(54) **VERFAHREN ZUR HERSTELLUNG VON SULFONSÄUREDIAMIDEN**
METHOD FOR THE PRODUCTION OF SULPHONIC ACID DIAMIDES
PROCEDE POUR LA PRODUCTION DE DIAMIDES D'ACIDE SULFONIQUE

(30) Priorität: 16.05.2002 DE 10221910
(43) Veröffentlichungstag der Anmeldung: 16.02.2005
(73) Patentinhaber: BASF SE, 67056 Ludwigshafen (DE)
(72) Erfinder: HAMPRECHT, Gerhard, 69469 Weinheim (DE); PUHL, Michael, 68623 Lampertheim (DE); REINHARD, Robert, 67065 Ludwigshafen (DE); SAGASSER, Ingo, 67454 Hassloch (DE); SCHMIDT, Thomas, 67433 Neustadt (DE); GÖTZ, Norbert, 67547 Worms (DE); ZIERKE, Thomas, 67459 Böhl-Iggelheim (DE)
(86) Internationale Anmeldenummer: PCT/EP2003/005126
(87) Internationale Veröffentlichungsnummer: WO 2003/097589

(56) Entgegenhaltungen:
- WO-A-01/83459
- DE-B- 1 028 129
- GB-A- 1 488 186
- GB-A- 2 038 334
- US-A- 4 049 709
- US-A- 4 131 620
- US-A- 4 260 560
- US-A- 4 868 308
- US-A- 5 099 025
- DATABASE CROSSFIRE BEILSTEIN [Online] Beilstein Institut zur Förderung der Chemischen Wissenschaften, Frankfurt am Main, DE; Database accession no. BRN 2362206 XP002253897 & R.SOWADA: J.PRAKT.CHEM., Bd. 33, 1966, Seiten 240-246,
- DATABASE CROSSFIRE BEILSTEIN [Online] Beilstein Institut zur Förderung der Chemischen Wissenschaften, Frankfurt am Main, DE; Database accession no. BRN 58 147 89 XP002253898 & J.DANIEL ET AL.: CHEM. LETT., Bd. 8, 1992, Seiten 1575-1578,
- DATABASE CROSSFIRE BEILSTEIN [Online] Beilstein Institut zur Förderung der Chemischen Wissenschaften, Frankfurt am Main, DE; Database accession no. BRN 1116113 XP002253899 & K. CLAUSS ET AL.: JUSTUS LIEBIGS ANN. CHEM., 1974, Seiten 561-592,
- DATABASE CROSSFIRE BEILSTEIN [Online] Beilstein Institut zur Förderung der Chemischen Wissenschaften, Frankfurt am Main, DE; Database accession no. BRN 991720 XP002253900 & L.SRIDHARA: Z.NATURFORSCH.B ANORG. CHEM. ORG. CHEM., Bd. 30, 1975, Seite 969
- DATABASE CROSSFIRE BEILSTEIN [Online] Beilstein Institut zur Förderung der Chemischen Wissenschaften, Frankfurt am Main, DE; Database accession no. BRN 14 57 235 XP002253901 & M.MEYER: TETRAHEDRON LETT., 1968, Seite 3823
- DATABASE CROSSFIRE BEILSTEIN [Online] Beilstein Institut zur Förderung der Chemischen Wissenschaften, Frankfurt am Main, DE; Database accession no. BRN 84 03 296 XP002253902 & BULL. CEHM. SOC. JPN, Bd. 72, Nr. 9, 1999, Seiten 2115-2116,

## Beschreibung

Die Erfindung betrifft ein Verfahren zur Herstellung von sulfonsäure diamiden.

Sulfamidsäurehalogenide primärer oder sekundärer Amine sind interessante Zwischenstufen für die Herstellung von Wirkstoffen mit Sulfonamid- oder Sulfonyldiamid-Struktureinheiten. Eine Übersicht über Alkylsulfamidsäurechloride, deren Herstellung und deren Verwendung findet man in G. Hamprecht et al. Angew. Chem. 93, (1981), S. 151-163.

Grundsätzlich kann man Sulfamidsäurechloride durch Umsetzung von Monoalkylammoniumchloriden mit Sulfonylchlorid, im Folgenden auch Sulfurylchlorid, herstellen (Acta Chem. Scand. 19, (1963), S. 2141 sowie DE-A 1242627). Nachteilig an diesem Verfahren sind die langen Reaktionszeiten. Zudem führt die Verwendung von Sulfonylchlorid zu einer Vielzahl von Nebenreaktionen. Bei langkettigen Aminen dominiert beispielsweise die chlorierende Wirkung des Sulfonylchlorids, so dass auf diesem Weg Sulfamylchloride derartiger Amine nicht zugänglich sind.

Von diesen Verfahren wird auch in der WO 98/28280, WO 00/18770, WO 01/64808 und Bull. Soc. Chim. Belg. 93, 1984, S. 920 für die Herstellung von N-Methoxyethyl-N-methylsulfamidsäurechlorid, N-Cyanoethyl-N-methylsulfamidsäurechlorid, N-Allyl-N-methylsulfamidsäurechlorid und Bis-N-allylsulfamidsäurechlorid Gebrauch gemacht. Die gefundenen Ausbeuten sind durchgängig niedrig.

Aus der DE-A 2164176 und der EP-A 11794 sind Verfahren zur Herstellung von Sulfamidsäurehalogeniden der Formel R-NH-SO₂X,
worin R für einen aliphatischen oder cycloaliphatischen Rest steht und X ein Halogenatom bedeutet, bekannt, bei dem man eine Sulfamidsäure der allgemeinen Formel R-NH-SO₃H, worin R die vorgenannte Bedeutung hat, mit einem Säurehalogenid des Phosphors umsetzt. Die als Ausgangsmaterialien eingesetzten Sulfamidsäuren werden durch Umsetzung von Isocyanaten R-N=C=0 mit Schwefelsäure oder durch Reaktion disubstituierter Harnstoffe mit Oleum hergestellt. Von Nachteil ist zum einen, dass diese Verfahren von vergleichsweise teuren und aufwendig herzustellenden Ausgangsprodukten ausgehen. Zudem ist dieses Verfahren aufgrund der für die Herstellung der Sulfamidsäuren erforderlichen Reaktionsbedingungen (Schwefelsäure bzw. Oleum) nur für die Herstellung solcher Sulfamidsäurehalogenide geeignet, die am Stickstoff einen vergleichsweise inerten Kohlenwasserstoffrest tragen. Dieses Verfahren ist nicht geeignet für die Herstellung von Sulfamidsäurehalogeniden, die reaktive Gruppen, z.B. olefinische Doppelbindungen oder Dreifachbindungen, Cyanoalkylgruppen, Alkoxyalkylgruppen oder aldehydische Carbonylgruppen aufweisen.

R. Wegler et al. beschreiben in J. Liebigs Ann. Chem. (1959), 624, S. 25-29 die Herstellung von N,N-Dialkylsulfamidsäurechloriden, indem man zunächst die sekundären Amine bzw. deren Hydrochloride durch Behandlung mit Chlor in die N-Chloramine überführt, welche anschließend durch Umsetzung mit Schwefeldioxid in Gegenwart von Chlor in die Sulfamidchloride überführt werden. Als Alternative hierzu wird die Umsetzung von Dialkylaminen mit Schwefeldioxid in Tetrachlorkohlenstoff mit nachfolgender Umsetzung der dabei erhaltenen Thioamidsäuren mit Chlor zu den Sulfamidsäurechloriden beschrieben. Nachteilig an beiden Verfahren ist die Verwendung von elementarem Chlor, wodurch das Verfahren auf solche Amine beschränkt ist, die keine gegenüber Chlor reaktive Gruppen aufweisen. Weiterhin wird in der ersten Verfahrensvariante intermediär ein sehr instabiles N-Chloramin erzeugt, das in seiner Handhabung sehr problematisch ist.

In dem deutschen Patent 946710 wird die Herstellung von Sulfamidsäurechloriden durch Umsetzung von Carbaminsäurechloriden mit Schwefeltrioxid beschrieben.

R.E. Olson et al., J. Med. Chem. (1999), 42, S. 1189 beschreibt die Herstellung von Isobutylsulfamidchlorid aus Isobutylamin, wobei man zunächst Isobutylamin mit Chlorsulfonsäure umsetzt und das dabei erhaltene Isobutylammoniumsalz der Isobutylsulfamid-Säure mit Phosphorpentachlorid umsetzt. Die dabei erhaltenen Ausbeuten sind jedoch nicht zufriedenstellend.

Obwohl eine Vielzahl von Verfahren zur Herstellung von Sulfamidsäurechloriden aus dem Stand der Technik bekannt sind, ist es bislang nicht gelungen, ein effizientes Verfahren zur Herstellung von Sulfamidsäurechloriden bereitzustellen, das nicht auf die Herstellung von Sulfamidsäurechloriden inerter Amine beschränkt ist, die keine der oben genannten reaktiven Gruppen aufweisen, und bei dem die Amine direkt als Ausgangsmaterialien eingesetzt werden können. Der vorliegenden Erfindung liegt somit die Aufgabe zugrunde, ein solches Verfahren bereitzustellen.

Es wurde überraschenderweise gefunden, dass diese Aufgabe durch ein Verfahren gelöst wird, bei dem man zunächst ein primäres oder sekundäres Amin mit wenigstens äquimolaren Mengen an Schwefeltrioxid oder einer Schwefeltrioxidquelle in Gegenwart wenigstens äquimolarer Mengen eines tertiären Amins A2 umsetzt und das dabei erhaltene Amidosulfonsäureammoniumsalz mit wenigstens der stöchiometrisch erforderlichen Menge eines Phosphorhalogenids umsetzt.

Die nach dem erfindungsgemäßen Verfahren erhaltenen Sulfamidsäurehalogenide werden in Analogie zu bekannten Methoden durch Umsetzung mit Ammoniak in die entsprechenden Sulfamidsäureamide (= Sulfonsäurediamide) umgewandelt. Die Umsetzung liefert die Verbindungen in hohen Ausbeuten. Derartige Sulfamidsäureamide wurden bislang durch Umsetzung von durch partielle Hydrolyse von Chlorsulfonsäureisocyanat zum Chlorsulfonsäureamid Cl-SO₂-NH₂ und anschließende Umsetzung des Chlorsulfonsäureamids mit einem primären oder sekundären Amin hergestellt (siehe z.B. WO 00/83459). Die Sulfamidsäureamide sind auf diesem Wege jedoch nur in mäßigen Ausbeuten von in der Regel < 50 % erhältlich. Zudem erfordert das Verfahren den Einsatz des hochreaktiven, sehr feuchtigkeitsempfindlichen und kostspieligen Chlorsulfonylisocyanats. Demnach betrifft die vorliegende Erfindung ein Verfahren zur Herstellung von Sulfonsäurediamiden durch:
i) Umsetzung eines primären oder sekundären Amins A1 mit wenigstens äquimolaren Mengen SO₃ oder einer SO₃-Quelle in Gegenwart von wenigstens äquimolaren Mengen eines tertiären Amins A2, jeweils bezogen auf das Amin A1, und
ii) Umsetzung der in Schritt i) erhaltenen Reaktionsmischung mit wenigstens der stöchiometrisch erforderlichen Menge eines Phosphorhalogenids und
iii)Umsetzung des in Schritt ii) erhaltenen Sulfamidsäurehalogenids mit Ammoniak.

Das erfindungsgemäße Verfahren zur Herstellung der Sulfodiamide liefert die entsprechenden Sulfonsäurediamide in sehr hohen Ausbeuten und ist daher Gegenstand der vorliegenden Erfindung. Eine Verwendung von Chlorsulfonylisocyanat ist in diesem Verfahren nicht erforderlich.

Das erfindungsgemäße Verfahren wird am besten durch die in Schema 1 gezeigte Reaktionsgleichung beschrieben.

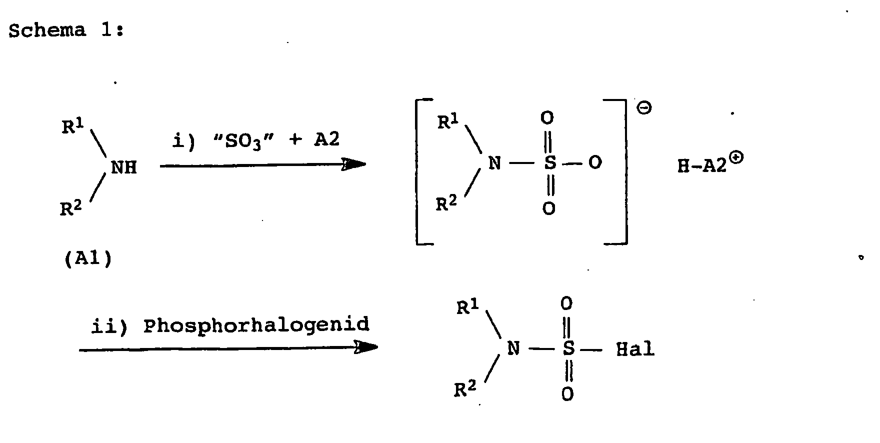

In Schema 1 steht R¹R²-NH für ein primäres oder sekundäres Amin A1, A2 steht für ein tertiäres Amin und Hal steht für ein Halogenatom, das aus dem Phosphorhalogenid übertragen wurde.

Beispiele für geeignete primäre oder sekundäre Amine sind solche der Formeln IA und IB

R¹-NH₂ (IA); R¹R²N-A (IB),

worin
- R¹ und R²: unabhängig voneinander für C₁-C₂₀-Alkyl, C₂-C₂₀-Alkenyl oder C₂-C₂₀-Alkinyl, die unsubstituiert oder durch CN, NO₂, C₁-C₄-Alkoxy, C₁-C₄-Alkylthio, Formyl, C₁-C₄-Alkylcarbonyl, C₁-C₄-Alkoxycarbonyl, C₁-C₄-Alkylaminocarbonyl, C₁-C₄-Dialkylaminocarbonyl, C₁-C₄-Alkylsulfinyl, C₁-C₄-Alkylsulfonyl, C₃-C₁₀-Cycloalkyl, Phenyl, das seinerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen kann, substituiert sein können,
C₁-C₂₀-Halogenalkyl, C₂-C₂₀-Halogenalkenyl, C₂-C₂₀-Halogenalkinyl, C₃-C₁₀-Cycloalkyl, C₅-C₁₀-Cycloalkenyl, Heterocyclyl mit ein bis drei Heteroatomen, ausgewählt unter O, S und N, Phenyl oder Naphthyl, wobei Heterocyclyl, Phenyl oder Naphthyl, ihrerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen können,
- R¹ und R²: auch gemeinsam einen gesättigten oder teilweise ungesättigten 5- bis 8-gliedrigen Stickstoffheterocyclus bilden können, der seinerseits durch C₁-C₄-Alkyl, C₁-C₄-Alkoxy und/oder C₁-C₄-Halogenalkyl, substituiert sein kann, ein oder 2 Carbonylgruppen, Thiocarbonylgruppen und/oder ein oder zwei weitere Heteroatome, ausgewählt unter O, S und N, als Ringglieder aufweisen kann,

Die hier und im Folgenden für die Substituenten oder als Reste an Phenyl- und Heterocyclyl-Resten genannten organischen Molekülteile stellen Sammelbegriffe für individuelle Aufzählungen der einzelnen Gruppenmitglieder dar, wobei der Ausdruck Cₙ-Cₘ die mögliche Anzahl der Kohlenstoffatome im Molekülteil angibt. Sämtliche Kohlenwasserstoffketten, also alle Alkyl-, Alkenyl- und Alkinylteile können geradkettig oder verzweigt sein. Sofern nicht anders angegeben tragen halogenierte Substituenten vorzugsweise ein bis sechs gleiche oder verschiedene Halogenatome. Die Bedeutung Halogen steht jeweils für Fluor, Chlor, Brom oder Iod.

Im einzelnen bedeutet beispielsweise:
- C₁-C₄-Alkyl für: z. B. Methyl, Ethyl, Propyl, 1-Methylethyl, Butyl, methylpropyl, 2-Methylpropyl oder 1,1-Dimethylethyl;
- C₁-C₂₀-Alkyl: ein gesättigter aliphatischer Kohlenwasserstoffrest mit 1 bis 20 C-Atomen und insbesondere mit 1 bis 10 C-Atomen (C₁-C₁₀-Alkyl) z.B. C₁-C₄-Alkyl, wie voranstehend genannt, sowie z. B. n-Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl, 1-Ethyl-3-methylpropyl, n-Heptyl, n-Nonyl, n-Decyl, 1-Methylhexyl, 1-Ethylhexyl, 1-Methylheptyl, 1-Methyloctyl, 1-Methylnonyl, n-Undecyl, 1-Ethylnonyl, 2-Ethylnonyl, 1,2-Dimethylnonyl, n-Dodecyl, 1-Methylundecyl, 1-Ethyldecyl, n-Tridecyl, 1-Methyldodecyl, 1-Ethylundecyl, n-Tetradecyl, 1-Methyltridecyl, 1-Ethyldodecyl, n-Pentadecyl, 1-Methyltetradecyl, 1-Ethyltridecyl, n-Hexadecyl, 1-Methylpentadecyl, 1-Ethyltetradecyl, n-Heptadecyl, 1-Methylhexadecyl, 1-Ethylpentadecyl, n-Octadecyl, 1-Methylheptadecyl, 1-Ethylhexadecyl, n-Nonadecyl, 1-Methyloctadecyl, n-Eicosyl, 1-Methylnonadecyl;
- C₂-C₂₀-Alkenyl: ein einfach ungesättigter olefinischer Kohlenwasserstoffrest mit 2 bis 20 C-Atomen, vorzugsweise 2 bis 10 und insbesondere 3 bis 6 C-Atomen (C₂-C₁₀-Alkenyl bzw. C₃-C₆-Alkenyl), z. B. Ethenyl, Prop-2-en-1-yl (= Allyl), Prop-1-en-1-yl, But-1-en-4-yl, But-2-en-1-yl, But-3-en-1-yl, 1-Methylprop-2-en-1-yl, 2-Methylprop-2-en-1-yl, 1-Penten-3-yl, 1-Penten-4-yl, 2-Penten-4-yl, 1-Methylbut-2-en-1-yl, 2-Methylbut-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-1-yl, 1-Ethylprop-1-en-2-yl, n-Hex-1-en-1-yl, n-Hex-2-en-1-yl, Hex-3-en-1-yl, Hex-4-en-1-yl, Hex-5-en-1-yl, 1-Methyl-pent-1-en-1-yl, 2-Methylpent-1-en-1-yl, 3-Methylpent-1-en-1-yl, 4-Methylpent-1-en-1-yl, 1-Methylpent-2-en-1-yl, 2-Methylpent-2-en-1-yl, 3-Methylpent-2-en-1-yl, 4-Methylpent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethylbut-2-en-1-yl, 1-Ethylbut-3-en-1-yl, 2-Ethylbut-2-en-1-yl, 2-Ethylbut-3-en-1-yl, 1,1,2-Trimethylprop-2-en-1-yl, 1-Ethyl-1-methylprop-2-en-7-yl, 1-Ethyl-2-methylprop-2-en-1-yl, Hept-2-en-1-yl, Oct-2-en-1-yl, Non-2-en-1-yl, Dec-2-en-1-yl, Undec-2-en-1-yl, Dodec-2-en-1-yl, Tridec-2-en-1-yl, Tetradec-2-en-1-yl, Pentadec-2-en-1-yl, Hexadec-2-en-1-yl, Heptadec-2-en-1-yl, Octadec-2-en-1-yl, Nonadec-2-en-1-yl, Eicosa-2-en-1-yl ;
   C₂-C₂₀-Alkinyl: ein Kohlenwasserstoffrest mit 2 bis 20 C-Atomen, vorzugsweise 2 bis 10 und insbesondere 3 bis 6 C-Atomen und einer Dreifachbindung (C₂-C₁₀-Alkinyl bzw. C₃-C₆-Alkinyl), z.B. Ethinyl, Prop-2-in-1-yl (= Propargyl), Prop-1-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methylbut-1-in-3-yl, 3-Methylbut-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methylpent-1-in-3-yl, 3-Methylpent-1-in-4-yl, 3-Methylpent-1-in-5-yl, 4-Methylpent-2-in-4-yl, 4--Methylpent-2-in-5-yl, Hept-2-in-1-yl, Oct-2-in-1-yl, Non-2-in-1-yl, Dec-2-in-1-yl, Undec-2-in-1-yl, Dodec-2-in-1-yl, Tridec-2-in-1-yl, Tetradec-2-in-1-yl, Pentadec-2-in-1-yl, Hexadec-2-in-1-yl, Heptadec-2-in-1-yl, Octadec-2-in-1-yl, Nonadec-2-in-1-yl, Eicosa-2-in-1-yl, ;
   C₁-C₄-Halogenalkyl für: einen C₁-C₄-Alkylrest, wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₂₀-Halogenalkyl: C₁-C₂₀-Alkyl, insbesondere C₁-C₁₀-Alkyl, wie vorstehend genannt, worin ein Teil oder alle und insbesondere 1 bis 6 Wasserstoffatome durch Halogenatome, vorzugsweise durch Fluor und/oder Chlor substituiert sind, z.B.: C₁-C₄-Halogenalkyl, wie vorstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₂-C₂₀-Halogenalkenyl: C₂-C₂₀-Alkenyl, insbesondere C₂-C₁₀-Alkenyl, wie vorstehend genannt, worin ein Teil oder alle und insbesondere 1 bis 6 Wasserstoffatome durch Halogenatome, vorzugsweise durch Fluor und/oder Chlor substituiert sind;
- C₂-C₂₀-Halogenalkinyl: C₂-C₂₀-Alkinyl, insbesondere C₂-C₁₀-Alkinyl, wie vorstehend genannt, worin ein Teil oder alle und insbesondere 1 bis 6 Wasserstoffatome durch Halogenatome, vorzugsweise durch Fluor und/oder Chlor substituiert sind;
- C₃-C₁₀-Cycloalkyl: für einen cycloaliphatischen Rest mit 3 bis 10 C-Atomen: z B. Cyclopropyl, Cyclobutyl, Cyclopentyl, Cyclohexyl, Cycloheptyl, Cyclooctyl, Cyclononyl oder Cyclodecyl;
- C₅-C₁₀-Cycloalkenyl: für einen cycloaliphatischen Rest mit 5 bis 10 C-Atomen, vorzugsweise 5 bis 8 C-Atomen und einer Doppelbindung: z B. Cyclopenten-1-yl, Cyclohexen-1-yl, Cyclohepten-1-yl, Cycloocten-1-yl, Cyclononen-1-yl, Cyclodecen-1-yl, Cyclopent-2-en-1-yl, Cyclohex-2-en-1-yl, Cyclohept-2-en-1-yl, Cyclooct-2-en-1-yl, cyclonon-2-en-1-yl, Cyclodec-2-en-1-yl, Cyclohex-3-en-1-yl, Cyclohept-3-en-1-yl, Cyclooct-3-en-1-yl, Cyclooct-4-en-1-yl, Cyclonon-3-en-1-yl, Cyclonon-4-en-1-yl, Cyclodec-4-en-1-yl oder Cyclodec-3-en-1-yl;
- C₁-C₁₀-Cyanoalkyl: durch eine CN-Gruppe substituiertes C₁-C₁₀-Alkyl, z. B Cyanomethyl, 1-Cyanoethyl, 2-Cyanoethyl, 1-Cyanopropyl, 2-Cyanopropyl, 3-Cyanopropyl, 1-Cyanoprop-2-yl, 2-Cyanoprop-2-yl, 1-Cyanobutyl, 2-Cyanobutyl, 3-Cyanobutyl, 4-Cyanobutyl, 1-Cyanobut-2-yl, 2-Cyanobut-2-yl, 1-Cyanobut-3-yl, 2-Cyanobut-3-yl, 1-Cyano-2-methylprop-3-yl, 2-Cyano-2-methylprop-3-yl, 3-Cyano-2-methylprop-3-yl, 3-Cyano-2,2-dimethylpropyl, 6-Cyanohex-1-yl, 7-Cyanohept-1-yl, 8-Cyanooct-1-yl, 9-Cyanonon-1-yl, 10-Cyanodec-1-yl;
- C₁-C₄-Alkylcarbonyl: für einen über eine Carbonylgruppe gebundenen Alkylrest mit 1 bis 4 C-Atomen, z.B. für Acetyl, Propionyl, Butyryl oder Isobutyryl;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di-(Ci-C4-alkyl)-aminocarbonyl: z. B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- C₁-C₄ Alkoxy: für einen über ein Sauerstoffatom gebundenen Alkylrest mit 1 bis 4 C-Atomen, z. B. Methoxy, Ethoxy, Propoxy, 1-Methylethoxy, Butoxy, 1-Methylpropoxy, 2-Methylpropoxy oder 1,1-Dimethylethoxy;
- C₁-C₄ Alkylthio (C₁-C₄-Alkylsulfanyl: C₁-C₄-Alkyl-S-): für einen über ein Schwefelatom gebundenen Alkylrest mit 1 bis 4 C-Atomen, z.B. Methylthio, Ethylthio, Propylthio, 1-Methylethylthio, Butylthio, 1-Methylpropylthio, 2-Methylpropylthio oder 1,1-Dimethylethylthio;
- C₁-C₄ Alkylsulfinyl (C₁-C₄-Alkyl-S(=O)-): z.B. für Methylsulfinyl, Ethylsulfinyl, Propylsulfinyl, 1-Methylethylsulfinyl, Butylsulfinyl, 1-Methylpropylsulfinyl, 2-Methylpropylsulfinyl oder 1,1-Dimethylethylsulfinyl;
- C₁-C₄-Alkylsulfonyl (C₁-C₄-Alkyl-S(=O)₂-): z. B. für Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl;

Die Bezeichnung Heterocyclyl umfasst sowohl gesättigte, teilweise ungesättigte als auch aromatische heterocyclische Reste.

Beispiele für aromatisches Heterocyclyl sind 2- und 3-Thienyl, 2-und 3-Furyl, 2- und 3-Pyrrolyl, 1-, 3- oder 4-Pyrazolyl, 2-, 3-oder 4-Pyridinyl, 2- oder 4-Oxazolyl und dergleichen.

Beispiele für gesättigte oder teilweise ungesättigte 5- bis 8-gliedrige stickstoffheterocyclische Reste, die ein oder 2 Carbonylgruppen, Thiocarbonylgruppen und/oder ein oder zwei weitere Heteroatome, ausgewählt unter O, S und N, als Ringglieder aufweisen können, sind Pyrrolidin-1-yl, 1,3-Oxazolidin-3-yl, 1,2-Oxazolidin-2-yl, 4,5-Dihydropyrazol-1-yl, Tetrahydropyrazol-1-yl, Piperidin-1-yl, Morpholin-4-yl, 2-Methylmorpholin-4-yl, 3-Methylmorpholin-4-yl, 2,6-Dimethylmorpholin-4-yl, Hexahydropyridazin-1-yl, Hexahydropyrimidin-1-yl, Hexahydropiperazin-1-yl, Hexahydro-1,3,5-triazin-1-yl, Hexahydroazepin-1-yl, Hexahydro-1,3-diazepin-1-yl, Hexahydro-1,4-diazepin-1-yl.

Bevorzugte Amine sind sekundäre Amine, d.h. R¹ und R² sind von Wasserstoff verschieden. Vorzugsweise weisen die Amine A1 nur eine primäre oder eine sekundäre Aminogruppe auf. Vorzugsweise weisen die Amine A1 keine alkoholischen Hydroxylgruppen auf.

Bevorzugte Substituenten R¹ und R² sind unabhängig voneinander ausgewählt unter C₁-C₁₀-Alkyl, C₃-C₁₀-Alkenyl, C₃-C₁₀-Alkinyl, worin die Doppel- bzw. die Dreifachbindung nicht direkt an dem Kohlenstoffatom angeordnet ist, das mit dem Stickstoffatom verbunden ist. Bevorzugt sind weiterhin C₁-C₄-Alkoxyalkyl, C₁-C₄-Alkylthio-C₁-C₄-alkyl, Cyano-C₁-C₄-alkyl, C₅-C₈-Cycloalkyl, C₅-C₈-Cycloalkinyl, Phenyl, das in der oben bezeichneten Weise und insbesondere durch Halogen oder C₁-C₄-Alkoxy substituiert sein kann. Vorzugsweise stehen nicht beide Reste R¹ und R² für gegebenenfalls substituiertes Phenyl, Naphthyl oder Heterocyclyl.

In bevorzugten Ausführungsformen können R¹ und R² mit dem Stickstoffatom, an das sie gebunden sind, einen gesättigten oder teilweise ungesättigten 5- oder 6-gliedrigen Stickstoffheterocyclus bilden, der in der oben genannten Weise substituiert sein kann, insbesondere 2,5-Dihydropyrrol-1-yl, 2,3-Dihydropyrrol-1-yl, 1-Pyrrolidinyl, 1-Piperidinyl, 4-Morpholinyl, 2-Methylmorpholin-4-yl, 2,6-Dimethylmorpholin-4-yl, 1-Methylpiperazin-4-yl.

Beispiele für geeignete Amine A1 sind die in der nachfolgenden Tabelle angegebenen Amine der Formel NR¹R², worin die Reste R¹ und R² jeweils die in einer Zeile der Tabelle 1 angegebene Bedeutung aufweisen:

**Tabelle 1:**

| | |
|---|---|
| R¹ | R² |
| CH₃ | CH₃ |
| CH₃ | C₂H₅ |
| CH₃ | CH(CH₃)₂ |
| CH₃ | CH₂CH=CH₂ |
| CH₃ | CH₂CH₂CN |
| CH₃ | CH₂CH₂CH₂CN |
| CH₃ | CH(CH₃)CH₂CN |
| CH₃ | CH₂CH(CH₃)CN |
| CH₃ | CH₂C≡CH |
| CH₃ | C₆H₅ |
| CH₃ | 2-Br-C₆H₄ |
| CH₃ | 3-Br-C₆H₄ |
| CH₃ | 4-Br-C₆H₄ |
| CH₃ | 2-Cl-C₆H₄ |
| CH₃ | 3-Cl-C₆H₄ |
| CH₃ | 4-Cl-C₆H₄ |
| CH₃ | C-C₃H₅ |
| CH₃ | C-C₅H₉ |
| CH₃ | CC₆H₁₁ |
| CH₃ | C-C₇H₁₃ |
| CH₃ | C-C₈H₁₅ |
| CH₃ | CH₂CH=CHCH₃ |
| CH₃ | CH(CH₃)C₂H₅ |
| CH₃ | CH₂CH(CH₃)₂ |
| CH₃ | CH₂CH₂CH₂Cl |
| CH₂CH₃ | CH₃ |
| CH₂CH₃ | C₂H₅ |
| CH₂CH₃ | CH(CH₃)₂ |
| CH₂CH₃ | CH₂CH=CH₂ |
| CH₂CH₃ | CH₂CH₂CN |
| CH₂CH₃ | CH₂CH₂CH₂CN |
| CH₂CH₃ | CH(CH₃)CH₂CN |
| CH₂CH₃ | CH₂CH(CH₃)CN |
| CH₂CH₃ | CH₂C≡CH |
| CH₂CH₃ | C₆H₅ |
| CH₂CH₃ | 2-Br-C₆H₄ |
| CH₂CH₃ | 3-Br-C₆H₄ |
| CH₂CH₃ | 4-Br-C₆H₄ |
| CH₂CH₃ | 2-Cl-C₆H₄ |
| CH₂CH₃ | 3-Cl-C₆H₄ |
| CH₂CH₃ | 4-Cl-C₆H₄ |
| CH₂CH₃ | C-C₃H₅ |
| CH₂CH₃ | C-C₅H₉ |
| CH₂CH₃ | C-C₆H₁₁ |
| CH₂CH₃ | C-C₇H₁₃ |
| CH₂CH₃ | C-C₈H₁₅ |
| CH₂CH₃ | CH₂CH=CHCH₃ |
| CH₂CH₂CH₃ | C₂H₅ |
| CH₂CH₂CH₃ | CH(CH₃)₂ |
| CH₂CH₂CH₃ | CH₂CH=CH₂ |
| CH₂CH₂CH₃ | CH₂CH₂CN |
| CH₂CH₂CH₃ | CH₂CH₂CH₂CN |
| CH₂CH₂CH₃ | CH(CH₃)CH₂CN |
| CH₂CH₂CH₃ | CH₂CH(CH₃)CN |
| CH₂CH₂CH₃ | CH₂C≡CH |
| CH₂CH₂CH₃ | C₆H₅ |
| CH₂CH₂CH₃ | 2-Br-C₆H₄ |
| CH₂CH₂CH₃ | 3-Br-C₆H₄ |
| CH₂CH₂CH₃ | 4-Br-C₆H₄. |
| CH₂CH₂CH₃ | 2-Cl-C₆H₄ |
| CH₂CH₂CH₃ | 3-Cl-C₆H₄ |
| CH₂CH₂CH₃ | 4-Cl-C₆H₄ |
| CH₂CH₂CH₃ | CH₂CH=CHCH₃ |
| CH(CH₃)₂ | CH₃ |
| CH(CH₃)₂ | C₂H₅ |
| CH(CH₃)₂ | CH(CH₃)₂ |
| CH(CH₃)₂ | CH₂CH=CH₂ |
| CH(CH₃)₂ | CH₂CH₂CN |
| CH(CH₃)₂ | CH₂CH₂CH₂CN |
| CH(CH₃)₂ 2 | CH(CH₃)CH₂CN |
| CH(CH₃)₂ | CH₂CH(CH₃)CN |
| CH(CH₃)₂ | CH₂CH=CHCH₃ |
| CH(CH₃)₂ | CH₂C≡CH |
| CH(CH₃)₂ | C₆H₅ |
| CH(CH₃)₂ | 2-Br-C₆H₄ |
| CH(CH₃)₂ | 3-Br-C₆H₄ |
| CH(CH₃)₂ | 4-Br-C₆H₄ |
| CH(CH₃)₂ | 2-Cl-C₆H₄ |
| CH(CH₃)₂ | 3-Cl-C₆H₄ |
| CH(CH₃)₂ | 4-Cl-C₆H₄ |
| CH₂CH=CH₂ | CH₂CH₂CN |
| CH₂CH=CH₂ | CH₂CH₂CH₂CN |
| CH₂CH=CH₂ | CH(CH₃)CH₂CN |
| CH₂CH=CH₂ | CH₂CH(CH₃)CN |
| H₅C₂OCH₂CH₂ | CH₃ |
| H₅C₂OCH₂CH₂ | C₂H₅ |
| H₅C₂OCH₂CH₂ | CH(CH₃)₂ |
| H₅C₂OCH₂CH₂ | CH₂CH=CH₂ |
| H₅C₂OCH₂CH₂ | CH₂CH₂CN |
| H₅C₂OCH₂CH₂ | CH₂CH₂CH₂CN |
| H₅C₂OCH₂CH₂ | CH(CH₃)CH₂CN |
| B₅C₂OCH₂CH₂ | CH₂CH(CH₃)CN |
| H₅C₂OCH₂CH₂ | CH₂C≡CH |
| H₅C₂SCH₂CH₂ | CH₃ |
| H₅C₂SCH₂CH₂ | C₂H₅ |
| H₅C₂SCH₂CH₂ | CH(CH₃)₂ |
| B₅C₂SCH₂CH₂ | CH₂CH=CH₂ |
| H₅C₂SCH₂CH₂ | CH₂CH₂CN |
| H₅C₂SCH₂CH₂ | CH₂CH₂CH₂CN |
| H₅C₂SCH₂CH₂ | CH(CH₃)CH₂CN |
| B₅C₂SCH₂CH₂ | CH₂CH(CH₃)CN |
| H₅C₂SCH₂CH₂ | CH₂C≡CH |
| H₃COCH₂CH₂ | CH₃ |
| H₃COCH₂CH₂ | C₂H₅ |
| H₃COCH₂CH₂ | CH(CH₃)₂ |
| H₃COCH₂CH₂ | CH₂CH=CH₂ |
| H₃COCH₂CH₂ | CH₂CH₂CN |
| H₃COCH₂CH₂ | CH₂CH₂CH₂CN |
| H₃COCH₂CH₂ | CH(CH₃)CH₂CN |
| H₃COCH₂CH₂ | CH₂CH (CH₃)CN |
| H₃COCH₂CH₂ | CH₂C≡CH |
| H₃CSCH₂CH₂ | CH₃ |
| H₃CSCH₂CH₂ | C₂H₅ |
| H₃CSCH₂CH₂ | CH(CH₃)₂ |
| H₃CSCH₂CH₂ | CH₂CH=CH₂ |
| H₃CSCH₂CH₂ | CH₂CH₂CN |
| H₃CSCH₂CH₂ | CH₂CH₂CH₂CN |
| H₃CSCH₂CH₂ | CH(CH₃)CH₂CN |
| H₃CSCH₂CH₂ | CH₂CH(CH₃)CN |
| H₃CSCH₂CH₂ | CH₂C≡CH |
| H₃COCH₂CH₂CH₂ | CH3 |
| H₃COCH₂CH₂CH₂ | C₂H₅ |
| H₃COµCH₂CH₂CH₂ | CH(CH₃)₂ |
| H₃COCH₂CH₂CH₂ | CH₂CH=CH₂ |
| H₃COCH₂CH₂CH₂ | CH₂CH₂CN |
| H₃COCH₂CH₂CH₂ | CH₂CH₂CH₂CN |
| H₃COCH₂CH₂CH₂ | CH(CH₃)CH₂CN |
| H₃COCH₂CH₂CH₂ | CH₂CH (CH₃) CN |
| H₃COCH₂CH₂CH₂ | CH₂C≡CH |
| B₃CSCH₂CH₂CH₂ | CH₃ |
| H₃CSCH₂CH₂CH₂ | C₂H₅ |
| H₃CSCH₂CH₂CH₂ | CH(CH₃)₂ |
| H₃CSCH₂CH₂CH₂ | CH₂CH=CH₂ |
| H₃CSCH₂CH₂CH₂ | CH₂CH₂CN |
| H₃CSCH₂CH₂CH₂ | CH₂CH₂CH₂CN |
| H₃CSCH₂CH₂CH₂ | CH(CH₃)CH₂CN |
| H₃CSCH₂CH₂CH₂ | CH₂CH(CH₃)CN |
| H₃CSCH₂CH₂CH₂ | CH₂C≡CH |
| | |
| CH₂-CH₂-O-CH₂-CH₂ | |
| CH₂-CH₂-CH₂-CH₂ | |
| CH₂-CH=CH-CH₂ | |
| CH=CH-CH₂-CH₂ | |
| CH₂-CH₂-CH₂-CH₂-CH₂ | |
| CH₂-CH₂-O-CH(CH₃)-CH₂ | |
| CH₂-CH₂-O-CH₂-CH(CH₃) | |
| CH₂-CH₂-N(CH₃)-CH₂-CH₂ | |
| CH₂-CH(CH₃)-O-CH(CH₃)-CH₂ | |
| CH₂-CH=CH-CH₂-CH₂ | |
| CH=CH-CH₂-CH₂-CH₂ | |
| CH₂-CH₂-CH₂-CH₂-CH(CH₃) | |
| CH₂-CH₂-CH₂-CH(CH₃)-CH₂ | |
| CH₂-CH₂-CH(CH₃)-CH₂-CH₂ | |
| CH₂-CH₂-CH₂-CH₂-CH(CH₂CH₂Cl) | |
| CH₂-CH₂-CH₂-CH(CH₂CH₂Cl)-CH₂ | |
| CH₂-CH₂-CH(CH₂CH₂Cl)-CH₂-CH₂ | |

Hinsichtlich der tertiären Amine A2 bestehen grundsätzlich keine Einschränkungen. Geeignet sind Trialkylamine, vorzugsweise Trialkylamine mit C₁-C₄-Alkylgruppen, wie Trimethylamin, Triethylamin, Dimethylethylamin, Dimethyl-n-propylamin, Tri-n-propylamin, Triisopropylamin, Tri-n-butylamin, Dimethyl-n-butylamin, N,N-Dialkyl-N-cycloalkylamine mit vorzugsweise C₁-C₄-Alkylgruppen und C₆-C₉-Cycloalkylgruppen, z.B. N,N-Dimethyl-N-cyclohexylamin, weiterhin N,N-Dialkylaniline mit vorzugsweise C₁-C₄-Alkylgruppen, insbesondere N,N-Dimethylanilin, N,N-Diethylanilin, N-Methyl-N-ethylanilin, heterocyclische tertiäre Amine, wie N-Alkylmorpholine, N-Alkylimidazole und N-Alkylpiperidine wie N-Methylmorpholin, N-Ethylmorpholin, N-Methylpiperidin, N-Ethylpiperidin, N-Ethylimidazol und N-Methylimidazol, weiterhin tertiäre Amine mit einem sp²-Stickstoffatom, die im Folgenden auch als tertiäre Amine vom Pyridintyp bezeichnet werden. Hierzu zählen neben Pyridin selber auch α-, β- und γ-Picolin, Pyrimidin, Pyridazin, 2,4-Lutidin, 2,6-Lutidin, Chinolin, Chinaldin sowie N-Alkyl-imidazole wie N-Methylimidazol und N-Ethylimidazol. Bevorzugte tertiäre Amine sind solche vom Pyridintyp, insbesondere Pyridin und α-, β- und γ-Picolin, besonders bevorzugt α-Picolin.

Als Schwefeltrioxidquelle kommen außer Schwefeltrioxid selber auch Chlorsulfonsäure und die Addukte von Schwefeltrioxid an die oben genannten tertiären Amine in Betracht. Unter den Addukten von Schwefeltrioxid an tertiäre Amine sind die Addukte an Picolin, Pyridin, Triethylamin, N,N-Dimethyl-N-cyclohexylamin bevorzugt. Diese Addukte lassen sich durch Zugabe von Schwefeltrioxid oder Chlorsulfonsäure zu einer Lösung des tertiären Amins in einem geeigneten Lösungsmittel, vorzugsweise dem Lösungsmittel der Reaktion herstellen. Die Zugabe erfolgt vorzugsweise im Bereich von -20 bis +50°C und insbesondere im Bereich von -10 bis +30°C.

Als Schwefeltrioxidquellen kommen weiterhin die Addukte von Schwefeltrioxid an sekundäre Amide wie Dimethylformamid, Diethylformamid, Di-n-propylformamid, Dimethylacetamid, Diethylacetamid, N-Methylacetanilid, N-Methylpyrrolidon, N-Ethylpyrrolidon, die Addukte an Tetraalkylharnstoffverbindungen wie Tetramethylharnstoff, Tetraethylharnstoff, Tetrabutylharnstoff und Dimethylpropylenharnstoff, weiterhin die Addukte an elektronenreiche Ether wie Tetrahydrofuran, Pyran oder die Addukte an Nitrile wie Acetonitril oder Propionitril in Betracht. Diese Addukte werden ähnlich wie die Addukte des Schwefeltrioxids an tertiäre Amine hergestellt. Bevorzugt wird in Schritt i) Schwefeltrioxid oder ein Schwefeltrioxid-Addukt an ein Amin vom Pyridintyp, besonders bevorzugt ein Schwefeltrioxid-Addukt an α-Picolin.

Erfindungsgemäß setzt man bei der Umsetzung des primären oder sekundären Amins A1 in Schritt i) wenigstens äquimolare Mengen, vorzugsweise wenigstens 1,1 und insbesondere wenigstens 1,2 Mol SO₃ oder SO₃-Addukt (gerechnet als SO₃) pro Mol Amin A1 ein. Zweckmäßigerweise wird man nicht mehr als 2,5 Mol und insbesondere nicht mehr als 2 Mol Schwefeltrioxid oder Schwefeltrioxid-Addukt pro Mol Amin A1 einsetzen.

An tertiärem Amin A2 wird man in Stufe i) vorzugsweise wenigstens 2 und insbesondere wenigstens 2,5 Mol pro Mol Amin A1 einsetzen. Zweckmäßigerweise beträgt die Menge an tertiärem Amin A2 nicht mehr als 6 Mol und insbesondere nicht mehr als 5 Mol pro Mol Amin A1. Im Falle, dass man als SO₃-Quelle das Addukt von SO₃ an ein tertiäres Amin einsetzt, wird die hierüber eingebrachte Menge an tertiärem Amin A2 bei den zuvor angegebenen Molverhältnissen mit berücksichtigt, d.h. die zuvor genannten Molangaben beziehen sich jeweils auf die Gesamtmenge an tertiärem Amin in Schritt i).

Üblicherweise erfolgt die Umsetzung in Schritt i) bei Temperaturen im Bereich von -20°C bis +100°C und vorzugsweise im Bereich von -10 bis + 60°C. Hierbei geht man in der Regel so vor, dass man das Schwefeltrioxid oder die Schwefeltrioxidquelle in einem geeigneten Lösungsmittel oder Verdünnungsmittel vorlegt und anschließend tertiäres Amin A2 zugibt. Die Zugabe erfolgt in der Regel im Bereich zwischen -20 und +100°C, vorzugsweise im Bereich von -10 bis +50°C und insbesondere im Bereich von -10 bis +20°C. Diese Reaktion ist häufig exotherm und wird in der Regel durch geeignete Maßnahmen der internen und/oder externen Kühlung in dem gewünschten Temperaturbereich gehalten. Sofern als SO₃-Quell ein Addukt von SO₃ an ein tertiäres Amin eingesetzt wird, ist die Zugabe des Amins zu der SO₃-Quell in der Regel weniger exotherm. Zu der hierbei erhaltenen Lösung bzw. Suspension gibt man dann das Amin A1. Die Zugabe des Amins A1 kann sowohl in Substanz als auch in gelöster bzw. suspendierter Form in einem hierfür geeigneten Lösungs- oder Verdünnungsmittel erfolgen. Hierbei bildet sich das in Schema 1 gezeigte Amidosulfonsäueammoniumsalz. Vorzugsweise erfolgt die Zugabe des Amins A1 bei Temperaturen im Bereich von -10 bis +60°C. Häufig läßt man im Anschluß an die Zugabe die erhaltene Reaktionsmischung noch eine Zeit nachreagieren, z.B. im Bereich von +10 bis +80°C und insbesondere im Bereich von +20 bis +60°C.

In einer anderen Ausführungsform der Erfindung führt man Schritt i) dergestalt durch, dass man das primäre oder sekundäre Amin A1 und das tertiäre Amin A2 in einem geeigneten Lösungs- oder Verdünnungsmittel vorlegt und hierzu SO₃ oder die SO₃-Quelle zugibt. Die Zugabe von SO₃ oder der SO₃-Quelle erfolgt ebenfalls in einem geeigneten Lösungs- oder Verdünnungsmittel. Für die Reaktionstemperaturen gilt das oben Gesagte. Bevorzugt erfolgt die Zugabe bei Temperaturen im Bereich von -20 bis +80°C und insbesondere im Bereich von -10 bis +60°C. Im Anschluß an die Zugabe läßt man die Reaktionsmischung noch eine Zeit nachreagieren. Hierbei betragen die Reaktionstemperaturen in der Regel +10 bis +80°C und vorzugsweise +20 bis +60°C.

Die für die Umsetzung in Schritt i) erforderliche Zeit beträgt in der Regel wenigstens 15 min und wird vorzugsweise 10 h und insbesondere 5 h nicht überschreiten.

Die Geschwindigkeit mit der das tertiäre Amin zu dem SO₃ bzw. der SO₃-Quell in der ersten Ausführungsform zugegeben wird, ist für das Ergebnis der Reaktion von untergeordneter Bedeutung und erfolgt in der Regel in einer Weise, die eine Temperaturkontrolle durch Kühlung erlaubt. Je nach Ansatzgröße liegt die Zeitdauer der Zugabe des Amins A2 im Bereich von wenigen Minuten bis zu 1 Stunde. Die Geschwindigkeit der Zugabe des Amins A1 zu der so erhaltenen Reaktionsmischung ist ebenfalls von untergeordneter Bedeutung und liegt häufig im Bereich von wenigen Minuten bis hin zu 1 Stunde. Zur Vervollständigung der Reaktion läßt man in der Regel wenige Minuten bis mehrere Stunden nachreagieren, z.B. 5 min bis 3 h. Häufig sind jedoch Reaktionsdauern von mehr als einer Stunde nicht erforderlich. Grundsätzlich ist es möglich, dass in Schritt i) erhaltene Amidosulfonsäureammoniumsalz zu isolieren und anschließend in Schritt ii) mit wenigstens der stöchiometrisch erforderlichen Menge eines Phosphorhalogenids umzusetzen. Vorzugsweise führt man jedoch die Umsetzung in Schritt ii) ohne vorherige Isolierung des Amidosulfonsäureammoniumsalzes aus der in Schritt i) erhaltenen Reaktionsmischung durch, d.h. man gibt das Phosphorhalogenid direkt zu der in Schritt i) erhaltenen Reaktionsmischung.

Als Phosphorhalogenide sind die üblichen kommerziell erhältlichen Phosphorhalogenide geeignet, insbesondere die Chloride und die Bromide und besonders bevorzugt die Chloride. Beispiele für geeignete Phosphorhalogenide sind Phosphortrichlorid, Phosphorpentachlorid, Phosphoroxychlorid, Phosphortribromid und Phosphorpentabromid. Bevorzugt sind Phosphortrichlorid, Phosphorpentachlorid und insbesondere Phosphoroxychlorid (= Phosphorylchlorid).

Das Phosphorhalogenid kann sowohl in Substanz als auch in einem inerten Lösungs- bzw. Verdünnungsmittel zugegeben werden. Im Falle flüssiger Phosphorhalogenide wie Phosphorylchlorid ist ein Lösen oder Verdünnen in der Regel nicht erforderlich.

Die erforderliche Mindestmenge an Phosphorhalogenid richtet sich in an sich bekannter Weise nach der Stöchiometrie der Reaktion und beträgt im Falle des Phosphorpentachlorids wenigstens 0,5 Mol je Mol Amin A1 und im Falle von Phosphortrichlorid, Phosphortribromid und Phosphoroxychlorid wenigstens 1 Mol je Mol Amin A1. Zweckmäßigerweise wird die Menge an Phosphorhalogenid 3 Mol je Mol Amin A1 und vorzugsweise 2,2 Mol je Mol Amin A1 nicht überschreiten. Bei Einsatz von Phosphortrichlorid, Phosphortribromid oder Phosphoroxychlorid beträgt die Menge an Phosphorhalogenid vorzugsweise 1 bis 3 Mol und insbesondere 1,1 bis 2,2 Mol je Mol Amin A1. Bei Verwendung von Phosphorpentachlorid oder Phosphorpentabromid beträgt die Menge vorzugsweise 0,5 bis 1 Mol, insbesondere 0,6 bis 0,9 Mol, pro Mol Amin A1.

Die für Schritt ii) erforderliche Reaktionszeit liegt in der Regel im Bereich von 0,5 bis 8 Stunden.

Die für Schritt ii) erforderlichen Reaktionstemperaturen liegen in der Regel im Bereich von 0 bis 100°C, vorzugsweise im Bereich von 10 bis 80°C und insbesondere im Bereich von 20 bis 80°C.

Geeignete Lösungs- bzw. Verdünnungsmittel für Schritt i) und ii) sind solche, die unter den Reaktionsbedingungen, d.h. gegenüber SO₃ und Phosphorhalogeniden inert sind. Derartige Lösungsmittel sind dem Fachmann bekannt und umfassen sowohl polare als auch unpolare aprotische Verbindungen wie cyclische oder offenkettige Ether, Halogenkohlenwasserstoffe, Nitrokohlenwasserstoffe, aromatische, aliphatische und cycloaliphatische Kohlenwasserstoffe, Tetraalkylharnstoffe, N-Alkyllactame, N,N-Dialkylamide und deren Mischungen. Beispiele für Ether sind Diethylether, Di-n-propylether, Methyl-tert.-butylether und Ethylenglycoldimethylether. Beispiele für Nitrokohlenwasserstoffe sind neben Nitromethan, Nitrobenzol, o-, m- oder p-Chlornitrobenzole, o-, p-Nitrotoluole. Beispiele für Kohlenwasserstoffe sind Benzol, Toluol, Xylole, Hexan, Heptan, Octan und Cyclohexan. Beispiel für Tetraalkylharnstoffe ist Tetramethylharnstoff. Beispiel für N,N-Dialkylamide ist Dimethylformamid und Dimethylacetamid. Ein Beispiel für N-Alkyllactame ist N-Methylpyrrolidon. Beispiele für Halogenkohlenwasserstoffe sind aliphatische Halogenkohlenwasserstoffe wie Methylenchlorid, 1,1- und 1,2-Dichlorethan, 1,2-cis-Dichlorethen, 1,1-, 1,2- und 1,3-Dichlorpropan, 1,4-Dichlorbutan, Tetrachlorethan, 1,1,1- und 1,1,2-Trichlorethan, Trichlorethylen, Pentachlorethan, Trichlorfluormethan, Chlorbenzol, Dichlorbenzole, Chlortoluole, Dichlortoluole, Trichlorbenzol und deren Mischungen. Bevorzugte Lösungsmittel sind Halogenkohlenwasserstoffe, insbesondere Dichlorethan, Dichlormethan und Chlorbenzol sowie deren Mischungen. Als Verdünnungsmittel kommen grundsätzlich auch die vorgenannten tertiären Amine in Betracht.

Die Menge an Verdünnungsmittel wird in der Regel so gewählt, dass die Reaktionsmischungen während der Umsetzung fließfähig bleiben, hierzu reichen in der Regel wenigstens 500 ml, vorzugsweise wenigstens 11 Lösungsmittel je Mol Amin A1 aus. Diese Angaben beziehen sich auf die Gesamtmenge an Lösungsmittel in den Reaktionsstufen i) und ii). Selbstverständlich wird man aus Kostengründen möglichst geringe Mengen an Lösungsmittel einsetzen. In der Regel wird daher die Lösungsmittelmenge nicht mehr als 5 l je Mol Amin A1 betragen.

Die Verfahrensschritte i) und ii) können sowohl diskontinuierlich als auch kontinuierlich in hierfür geeigneten Reaktionsgefäßen betrieben werden. Bei diskontinuierlicher Vorgehensweise wird man üblicherweise Rührkessel und Rührreaktoren einsetzen. Diese sind in der Regel zur Abfuhr der Reaktionswärme mit geeigneten Wärmetauschern oder einer Mantelkühlung ausgestattet. Die kontinuierliche Durchführung der Reaktionsschritte i) und ii) erfolgt ebenfalls in den hierfür üblichen Reaktoren, beispielsweise in Rührkesseln, Rührkesselkaskaden und Rohrreaktoren, wobei Reaktoren mit geringer Rückvermischung bevorzugt sind.

Die Aufarbeitung der in Schritt ii) erhaltenen Reaktionsmischung erfolgt in an sich üblicher Weise. Häufig wird man zur Zersetzung von überschüssigem Phosphorhalogenid die in Schritt ii) erhaltene Reaktionsmischung durch Eingießen in Wasser hydrolysieren und überschüssiges Amin A1 bzw. A2 nach Zugabe von verdünnter Säure, insbesondere verdünnter Mineralsäure, extraktiv mit einem organischen Lösungsmittel, das mit Wasser nicht mischbar ist, entfernen. Ebenfalls ist es möglich, überschüssiges Phosphorhalogenid und Lösungsmittel sowie gegebenenfalls überschüssiges tertiäres Amin A2 abzudestillieren und anschließend den Rückstand fraktioniert zu destillieren. Auch ist es möglich nach destillativer Entfernung flüchtiger Bestandteile den erhaltenen Rückstand mit einem organischen, mäßig polaren oder unpolaren Lösungsmittel zu versetzen, in dem Salze tertiärer Amine schlecht löslich sind. Geeignete Lösungsmittel sind offenkettige Ether, insbesondere Diethylether, Diisopropylether und Methyl-tert.-butylether. Auf diese Weise erhält man eine Lösung des Sulfamid-Säurehalogenids.

Nach Verdampfen des Lösungsmittels fällt das Sulfamidsäurehalogenid in der Regel in hinreichender Reinheit an, die seine direkte Verwendung in der Herstellung von Pflanzenschutzmitteln ermöglicht, ohne dass weitere Reinigungsstufen erforderlich sind. Die ohne Destillation erreichten Reinheiten liegen häufig bei 90 % und insbesondere bei 95 % und darüber. Aus diesem Grunde kann im erfindungsgemäßen Verfahren vorteilhafterweise auf eine Destillation verzichtet werden. Eine Destillation ist selbstverständlich jedoch grundsätzlich möglich.

Das erfindungsgemäße Verfahren liefert die Sulfamidsäurehalogenide in sehr guten Ausbeuten von in der Regel wenigstens 80 % und häufig wenigstens 90 %, bezogen auf das Amin A1.

Das erfindungsgemäße Verfahren erfordert zudem nicht den Umweg über Carbamidsäuren oder Isocyanate und umgeht auch den problematischen Einsatz von Chlor sowie die Herstellung von Chloraminen.

Das erfindungsgemäße Verfahren ermöglicht grundsätzlich die Herstellung beliebiger Sulfamidhalogenide, die sich von primären oder sekundären Aminen ableiten. Dabei können auch solche Amine eingesetzt werden, die vergleichsweise reaktive Funktionalitäten, insbesondere C=C-Doppelbindungen, C≡C-Dreifachbindungen, Aldehyd- oder Ketocarbonylgruppen, Ethergruppen, Estergruppen, Amidgruppen und dergleichen aufweisen. Insbesondere betrifft die vorliegende Erfindung die Herstellung von Sulfamidsäurehalogeniden der allgemeinen Formel II

Cl-SO₂-NR^{1'}R²' (II),

worin
- R^{1'}: für C₂-C₂₀-Alkenyl, C₂-C₂₀-Alkinyl, C₂-C₂₀-Halogenalkenyl, C₂-C₂₀-Halogenalkinyl, C₂-C₂₀-Alkyl, das durch CN, C₁-C₄-Alkoxy, C₂-C₂₀-Halogenalkenyl, C₂-C₂₀-Halogenalkinyl, C₅-C₁₀-Cycloalkenyl, C₁-C₄-Alkylcarbonyl, C₁-C₄-Alkoxycarbonyl, C₁-C₄-Alkylaminocarbonyl, C₁-C₄-Dialkylaminocarbonyl oder Formyl substituiert ist, oder C₅-C₁₀-Cycloalkenyl steht, und
- R^{2'}: C₂-C₂₀-Alkyl, C₂-C₂₁-Alkenyl oder C₂-C₂₀-Alkinyl bedeutet, die unsubstituiert oder durch CN, NO₂, C₁-C₄-Alkoxy, C₁-C₄-Alkylthio, Formyl, C₁-C₄-Alkylcarbonyl, C₁-C₄-Alkoxycarbonyl, C₁-C₄-Alkylaminocarbonyl, C₁-C₄-Dialkylaminocarbonyl, C₁-C₄-Alkylsulfinyl, C₁-C₄-Alkylsulfonyl, C₃-C₁₀-Cycloalkyl, Phenyl, das seinerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen kann, substituiert sein können, oder für
C₁-C₂₀-Halogenalkyl, C₂-C₂₀-Halogenalkenyl, C₂-C₂₀-Halogenalkinyl, C₃-C₁₀-Cycloalkyl, C₅-C₁₀-Cycloalkenyl, Heterocyclyl mit ein bis 3 Heteroatomen, ausgewählt unter O, S und N, Phenyl oder Naphthyl, wobei Heterocyclyl, Phenyl oder Naphthyl, ihrerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen können, steht und speziell C₁-C₄-Alkyl, Allyl, Propargyl oder Phenyl bedeutet, das unsubstituiert oder durch Halogen, Methoxy oder Methyl substituiert ist;
- R^{1'}: und R^{2'} auch gemeinsam einen teilweise ungesättigten 5- bis 8-gliedrigen Stickstoffheterocyclus bilden können, der seinerseits durch C₁-C₄-Alkyl, C₁-C₄-Alkoxy und/oder C₁-C₄-Halogenalkyl, substituiert sein kann, ein oder 2 Carbonylgruppen, Thiocarbonylgruppen und/oder ein oder zwei weitere Heteroatome, ausgewählt unter O, S und N, als Ringglieder aufweisen kann
wobei R^{2'} auch für Methyl stehen kann, wenn R^{1'} für C₂-C₂₀-Alkinyl, C₂-C₂₀-Halogenalkenyl, C₂-C₂₀-Alkyl, das durch C₂-C₂₀-Halogenalkenyl, C₂-C₂₀-Halogenalkinyl, C₅-C₁₀-Cycloalkenyl oder Formyl substituiert ist, oder C₅-C₁₀-Cycloalkenyl steht,
ausgenommen Verbindungen der allgemeinen Formel II, worin R^{1'} und R^{2'} jeweils für Allyl stehen.

Eine bevorzugte Ausführungsform der Erfindung betrifft Sulfamidsäurechloride der allgemeinen Formel II, worin R^{1'} und R^{2'} unabhängig voneinander und vorzugsweise in Kombination die nachfolgend angegebenen Bedeutungen aufweisen:
- R^{1'}: insbesondere C₁-C₄-Alkoxy-C₂-C₄-alkyl, Cyano-C₂-C₄-alkyl, C₃-C₁₀-Alkenyl, C₃-C₁₀-Alkinyl oder C₃-C₁₀-Halogenalkenyl, und speziell 2-Methoxyethyl, 2-Cyanoethyl, Allyl, Propargyl, 2-Chlorallyl steht;
- R^{2'}: C₂-C₄-Alkyl, Allyl, Propargyl oder Phenyl, das unsubstituiert oder durch Halogen, Methoxy oder Methyl substituiert ist, oder
- R^{1'}: und R^{2'} gemeinsam mit dem Stickstoffatom, an das sie gebunden sind, für 2,5-Dihydropyrrol-1-yl, 2,3-Dihydropyrrol-1-yl, 1,2,3,4- oder 1,2,3,6-Tetrahydropyridin-1-yl stehen, wobei die Heterocyclen 1, 2 oder 3 Methylgruppen aufweisen können.

Eine weitere Ausführungsform der Erfindung betrifft Sulfamidsäurechloride der allgemeinen Formel II, worin
- R^{1'}: für Phenyl oder Naphthyl steht, die 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen, und R^{2'} C₁-C₁₀-Alkyl oder C₃-C₁₀-Cycloalkyl bedeutet.

Die nach dem erfindungsgemäßen Verfahren erhaltenen Sulfonsäurediamide, insbesondere solche der allgemeinen Formel III

NH₂-SO₂-NR¹R² (III),

worin R¹ und R² die zuvor genannte Bedeutungen aufweisen, sind zur Herstellung von Verbindungen der allgemeinen Formel IV, 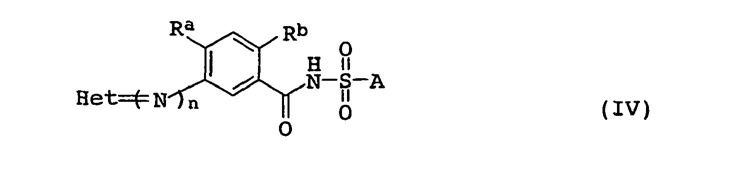 wie sie in der WO 01/83459 beschrieben sind, geeignet. In Formel IV steht n für 0 oder 1, Het bedeutet einen gegebenenfalls substituierten 5- oder 6-gliedrigen Heterocyclus, der 1, 2 oder 3 Stickstoffatome, gegebenenfalls ein weiteres Heteroatom ausgewählt unter Sauerstoff und Schwefel, und gegebenenfalls ein oder zwei Carbonyl- oder Thiocarbonylgruppen als Ringglieder aufweisen kann. R^{a} steht für Wasserstoff, Fluor oder Chlor, R^{b} für Chlor oder Cyano und A für einen von einem primären oder sekundären Amin A1 abgeleiteten Rest, insbesondere für NHR³ oder NR¹R², wobei R¹ und R² die zuvor genannten Bedeutungen aufweisen und R³ die für R¹ genannten Bedeutungen besitzt.

Demnach betrifft die vorliegende Erfindung auch ein Verfahren zur Herstellung von Verbindungen der allgemeinen Formel II. Dieses Verfahren umfasst die folgenden Schritte:
i) Umsetzung eines primären oder sekundären Amins A1 mit wenigstens äquimolaren Mengen SO₃ oder einer SO₃-Quelle in Gegenwart von wenigstens äquimolaren Mengen eines tertiären Amins A2, jeweils bezogen auf das Amin A1, wobei man ein Amidosulfonsäureammoniumsalz erhält;
ii) Umsetzung des Amidosulfonsäureammoniumsalz mit wenigstens der stöchiometrisch erforderlichen Menge eines Phosphorhalogenids, wobei man ein Sulfamidsäurehalogenid des Amins A1 erhält;
iii)Umsetzung des in Schritt ii) erhaltenen Sulfamidsäurehalogenids mit Ammoniak, wobei man ein Sulfamidsäureamid erhält; und
iv) Umsetzung des Reaktionsprodukts aus Schritt iii) mit einer Verbindung der allgemeinen Formel V 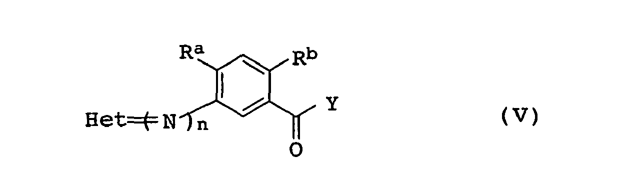 worin Het, n, R^{a} und R^{b} die für Formel IV angegebenen Bedeutungen aufweisen und Y für OH, Alkoxy oder ein Halogenatom steht.

Beispiele für geeignete Heterocyclen sind die in WO 01/83459 angegebenen Reste der Formeln Q1 bis Q40, auf die hiermit Bezug genommen wird. Vorzugsweise steht Het(=N)ₙ- für Reste der in WO 01/83459 angegebenen Formeln Q5, Q7, Q12, Q13, Q21, Q22, Q27, Q32, Q36, Q38, Q39, und Q40, z.B. für gegebenenfalls substituiertes Pyrimidin-2,6-dion-1-yl wie 4-Trifluormethylpyrimidin-2,6-dion-1-yl, 3-Methyl-4-trifluormethylpyrimidin-2,6-dion-1-yl, 3-Amino-4-trifluormethylpyrimidin-2,6-dion-1-yl, gegebenenfalls substituiertes 1,2,4-Triazol-5-on-1-yl wie 3-Methyl-4-difluormethyl-1,2,4-triazol-5-on-1-yl, gegebenenfalls substituiertes 1,3,5-Triazin-4,6-dion-5-yl wie 1,3-Dimethyl-2-thio-1,3,5-triazin-4,6-dion-5-yl oder 3,5-Dimethyl-1,3,5 -triazin-2,4,6-trion-1-yl, gegebenenfalls substituiertes 1,2,4-Triazin-6-yl wie 2,4-Dimethyl-3-thio-1,2,4-triazin-5-on-6-yl, gegebenenfalls substituiertes Pyrazin-3-on-2-yl wie 5-Trifluormethylpyrazin-3-on-2-yl, 4-Methyl-5-trifluormethylpyrazin-3-on-2-yl oder 4-Amino-5-methylsulfonylpyrazin-3-on-2-yl, gegebenenfalls substituiertes Pyrazol wie 4-Chlor-1-methyl-5-difluormethoxypyrazol-3-yl, 4-Brom-1-methyl-5-difluormethoxypyrazol-3-yl, 4-Chlor-1-methyl-5-trifluormethylpyrazol-3-yl oder 4-Brom-1-methyl-5-trifluormethylpyrazol-3-yl, gegebenenfalls substituiertes Pyridinyl wie 3-Chlor-5-trifluormethylpyridin-2-yl, 3,4,5,6-Tetrahydrophthalimidyl, für einen Rest der Formel: 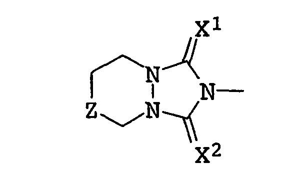 worin X¹, X² und Z unabhängig voneinander für Sauerstoff oder Schwefel stehen, und insbesondere für einen Rest der allgemeinen Formel 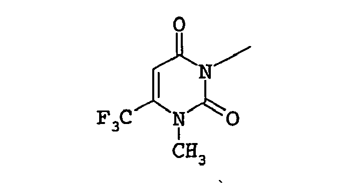 wenn n = 0 ist.

Hinsichtlich der Schritte i) und ii) gilt das oben Gesagte. Die Durchführung von Schritt iii) erfolgt in der Regel, indem man das Sulfamidsäurehalogenid, vorzugsweise das Chlorid, in einem geeigneten Lösungs- oder Verdünnungsmittel mit NH₃ oder einer wässrigen Ammoniak-Lösung umsetzt. Als Lösungs- oder Verdünnungsmittel sind neben den vorgenannten Lösungsmitteln insbesondere Wasser und mit Wasser mischbare Lösungs- und Verdünnungsmittel geeignet. Vorzugsweise erfolgt die Umsetzung in wässrigem Ammoniak, insbesondere in 5 bis 35 gew.-%igem wässrigem Ammoniak.

Vorzugsweise geht man so vor, dass man das Sulfamidsäurehalogenid, gegebenenfalls verdünnt in einem inerten Lösungsmittel, zu der Lösung des NH₃ in einem Lösungsmittel, vorzugsweise zu einer wässrigen Ammoniak-Lösung, gibt. Selbstverständlich kann man auch das Sulfamidsäurehalogenid, vorzugsweise in einem Lösungs- oder Verdünnungsmittel vorlegen und hierzu gasförmiges NH₃ oder eine Lösung von NH₃ in einem Lösungsmittel, insbesondere wässriges Ammoniak geben. Vorzugsweise wird NH₃ im Überschuss, bezogen auf die Stöchiometrie der Reaktion eingesetzt. Insbesondere wird man wenigstens 2,5 mol NH₃, z.B.-2,5 bis 50 Mol NH₃, insbesondere 3 bis 20 Mol NH₃, pro Mol Sulfamidsäurehalogenid einsetzen.

Die für die Umsetzung erforderlichen Temperaturen liegen in der Regel im Bereich von -20 bis 100°C und vorzugsweise im Bereich von -10 bis 30°C. Die Reaktionsdauer liegt in der Regel im Bereich von 10 min. bis 5 h und vorzugsweise im Bereich von 0,5 h bis 3 h.
Die Aufarbeitung des bei der Reaktion erhaltenen Sulfamidsäureamids erfolgt in an sich üblicher Weise, z.B. durch Entfernen des Lösungsmittels und Abtrennen der bei der Reaktion anfallenden Salze.

Schritt iv) wiederum erfolgt in an sich bekannter Weise, z.B. wie in der WO 01/83459, S. 31-35 beschrieben, indem man die Verbindung der Formel V mit den stöchiometrisch erforderlichen Mengen des gemäß Stufe iii) aus dem entsprechenden Sulfamidsäurechlorid erhaltenen Sulfamidsäureamids umsetzt.

Sofern Y für OH steht, erfolgt die Umsetzung beispielsweise in Gegenwart Wasser entziehender Mittel wie N,N'-Carbonyldiimidazol oder Dicyclohexylcarbodiimid in einem inerten organischen Lösungsmittel, wobei man gegebenenfalls zur Beschleunigung der Reaktion die Umsetzung in Gegenwart eines tertiären Amins oder einer Amidin-Base wie DBU (1,8-Diazabicyclo[5.4.0]undec-7-en] oder DBN (1,5-Diazabicyclo[4.3.0]non-5-en durchführt. Alternativ kann man auch Verbindungen V mit Y = OH zunächst in ihre Säurehalogenide überführen und anschließend mit dem Sulfamidsäureamid umsetzen. Derartige Umsetzungen sind dem Fachmann im Prinzip bekannt, z.B. aus Houben Weyl, Methoden der Organischen Chemie, Vol. E5 (1985), Teil I, S. 587 ff. und Vol. E5 (1985), Teil II S. 934 ff. Wegen weiterer Details wird an dieser Stelle außerdem auf die WO 01/83459 verwiesen.

Die folgenden Beispiele sollen die Erfindung verdeutlichten.

### I. Herstellung von Sulfamidsäurehalogeniden

### Beispiel 1

### N-(2-Chlor-2-propen-1-yl)-N-(n-propyl)-sulfamidsäurechlorid

35,7 g (0,256 mol) Schwefeltrioxid gab man als 57,4 %ige Lösung in 1,2-Dichlorethan innerhalb 15 min unter Rühren bei 0 bis 5°C zu einer Lösung von 43,7 g (0,469 mol) α-Picolin in 200 ml 1,2-Dichlorethan, spülte mit 40 ml 1,2-Dichlorethan nach und rührte 15 min bis zum Anstieg der Temperatur auf 20°C nach. Anschließend gab man 28,5 g (0,192 mol) 90 %iges N-(2-Chlor-2-propen-l-yl)-N-propylamin innerhalb von 15 min unter Rühren und äußerer Kühlung bei 20 bis 30°C zu, spülte mit 40 ml 1,2-Dichlorethan nach und rührte 15 min bei 50°C nach. Nach dem Abkühlen auf 23°C gab man 39,3 g (0,256 mol) Phosphoroxychlorid unter Rühren innerhalb 15 min zu, spülte mit 120 ml 1,2-Dichlorethan nach und erwärmte auf 70°C. Nach 1 h Rühren wurde das Reaktionsgemisch abgekühlt, im Vakuum eingeengt und der erhaltene Rückstand wurde mit je 100 ml Methyltert.-butylether verrührt. Die Methyl-tert.-butylether-Phasen wurden abdekantiert und eingeengt. Der Rückstand wurde destilliert. Man erhielt 42,5 g (90,5 % d. Th.) der Titelverbindung mit einem Kp. 67-71°C/0,4 mbar.

### Beispiel 2

### N-Methyl-N-[1-methylethyl]sulfamidsäurechlorid

63,2 g (0,41 mol) Schwefeltrioxid gab man als 52 %ige Lösung in 1,2-Dichlorethan innerhalb 15 min unter Rühren bei 0 bis 5°C zu einer Lösung von 70,0 g (0,752 mol) α-Picolin in 250 ml 1,2-Dichlorethan, spülte mit 50 ml 1,2-Dichlorethan nach und rührte 15 min bis zum Anstieg der Temperatur auf 25°C nach. Anschließend gab man hierzu 26,3 g (0,342 mol) 95 %iges N-Methyl-N-[1-methylethyl]amin innerhalb von 15 min unter Rühren bei 20 bis 35°C zu, spülte mit 50 ml 1,2-Dichlorethan nach und rührte 15 min bei 55°C. Nach dem Abkühlen auf 20°C gab man 42,7 g (0,205 mol) Phosphorpentachlorid unter Rühren innerhalb 15 min bei 20 bis 32°C und äußerer Kühlung zu und spülte mit 150 ml 1,2-Dichlorethan nach. Nach 2 h Rühren bei 70°C wurde das Reaktionsgemisch im Vakuum eingeengt und über einen Normag-Kolonnenkopf mit 10 cm Kolonne destilliert. Man erhielt 35 g (59,6 % d. Th.) der Titelverbindung mit einem Kp. von 110-115°C/30 mbar. Brechungsindex n_{D}²³=1,4620.

### Beispiel 3

### N-Isopropyl-N-(n-propyl)-sulfamidsäurechlorid

52,6 g (0,356 mol) Schwefeltrioxid gab man als 60 Gew.-%ige Lösung in 1,2-Dichlorethan innerhalb 25 min unter Rühren bei 0 bis 5°C zu einer Lösung von 60,75 g (0,652 mol) α-Picolin in 400 ml 1,2-Dichlorethan, spülte mit 80 ml 1,2-Dichlorethan nach und rührte 15 min bis zum Anstieg der Temperatur auf 22°C nach. Anschließend gab man 30 g (0,296 mol) N-Isopropyl-N-(n-propyl)amin innerhalb von 20 min unter Rühren und äußerer Kühlung bei 20 bis 30°C zu, spülte mit 80 ml 1,2-Dichlorethan nach und rührte 15 min bei 50°C nach. Nach dem Abkühlen auf 25°C gab man 54,6 g (0,356 mol) Phosphoroxychlorid unter Rühren und Kühlen auf 25-30°C innerhalb 15 min zu, spülte mit 200 ml 1,2-Dichlorethan nach und erwärmte auf 75°C. Nach 1 h Rühren bei dieser Temperatur wurde das Reaktionsgemisch abgekühlt, im Vakuum eingeengt und der erhaltene Rückstand wurde 3 mal mit je 200 ml Methyl-tert.-butylether verrührt. Die Methyl-tert.-butylether-Phasen wurden abdekantiert und 2 mal mit verdünnter Salzsäure extrahiert. Man trocknete die organische Phase über Magnesiumsulfat, filtrierte das Trockenmittels ab und engte ein. Man erhielt 55,1 g (91,3 % d. Th.) der Titelverbindung mit einem Brechungsindex n_{D}²³ = 1,4605. Eine gaschromatographische Untersuchung (Säule: 25 m Optima 17 GC 9 der Fa. Macherey und Nagel; Druck 14,5 psi; Helium; Säulenfluss 0,6 ml/min; Split, 30 ml/min; Injektor 280°C, Detektor 320°C) zeigte einen Reinheitsgrad von 96 % an (RT = 11,87 min).

### Beispiel 13

### N-Allyl-N-(2-cyanoethyl)-sulfamidsäurechlorid

64,8 g (0,641 mol) Schwefeltrioxid gab man als 57%ige Lösung in 1,2-Dichlorethan innerhalb 15 Minuten unter Rühren bei 0 bis 5°C zu einer Lösung von 90,6 g (0,846 mol) 2,6-Lutidin in 200 ml 1,2-Dichlorethan, spülte mit 40 ml 1,2-Dichlorethan nach und rührte 15 Minuten bis zum Anstieg der Temperatur auf 22°C nach. Anschließend gab man 42,4 g (0,385 mol) N-Allyl-N-(2-cyanoethyl)-amin innerhalb 15 Minuten unter Rühren bei 20 bis 30°C zu, spülte mit 40 ml 1,2-Dichlorethan nach und rührte 15 Minuten bei 50°C nach. Nach dem Abkühlen auf 22°C gab man 70,8 g (0,61 mol) Phosphoroxychlorid unter Rühren bei 20 bis 30°C innerhalb 15 Minuten zu, spülte mit 120 ml 1,2-Dichlorethan nach und erwärmte auf 70°C. Nach 1 Stunde Rühren bei dieser Temperatur ließ man das Reaktionsgemisch auf 25°C abkühlen, engte im Vakuum ein und destillierte über einen Normag-Kolonnenkopf mit 10 cm Kolonne. Man erhielt 32,3 g (40 % d. Th.) der Titelverbindung mit einem Kp. von 110 bis 116°C/0,4mbar. Brechungsindex: n_{D}²³ = 1,4948.

Analog Beispiel 1 wurden die in Tabelle 2 beschriebenen Sulfamidchloride R¹R²N-SO₂-CL der Beispiele 4 bis 24 hergestellt. In Tabelle 2 sind Siedepunkt [Kp in °C], der Brechungsindex [n_{D}²³ bzw. n_{D}²⁵], bei GC-Analytik (s.u.) die Retentionszeit RT der GC (in min) und der Reinheitsgrad, sowie die Ausbeute angegeben.

**Tabelle 2:**

| Bsp | R¹ | R² | Kp.[°C]/mbar oder RT [min] ; Reinheit 1) | n_{D}²³ (*= n_{D}²⁵) | Ausbeute [%] |
|---|---|---|---|---|---|
| 4 | C₂H₅ | n-C₃H₇ | 45-46/0,3 | 1,4599 | 89 |
| 5 | C₂H₅ | i-C₃H₇ | 49-52/0,3 | 1,4627 | 72,5 |
| 6 | CH₂-CH=CH-CH₂ -CH₂ | | 58-60 / 0,4 | 1,5102* | 88,5 |
| 7 | CH₂-CH(CH₃)-CH₂-CH₂-CH₂ | | 67-72 / 0,3 | 1,4908 | 78,8 |
| 8 | CH₂-CH(CH₃)-O- CH₂-CH₂ | | 61-64 / 0,2 | 1,4932 | 74,6 |
| 9 | CH(CH₂CH2Cl)-C CH₂-CH₂-CH₂-CH₂ | | 120-122 / 0,3 | 1,5172 | 75 |
| 10 | n-C₃H₇ | CH₃O(CH₂)₂ | 63-70/0,4 | 1,4619* | 85,5 |
| 11 | n-C₃H₇ | CH₂=CH-CH₂- | 11,52; 96 % | 1,4695 | 79,4 |
| 12 | C-C₆H₁₁ | CH₂=CH-CH₂- | | | |
| 13 | NC(CH₂)₂ | CH₂=CH-CH₂ | Kp. 110-116/0,4 | 1,4948 | 40 ²⁾ |
| 14 | NC(CH₂)₂ | EtS(CH₂)₂ | | | |
| 15 | NC(CH₂)₂ | C₂H₅ | | | |
| 16 | NC(CH₂)₂ | i-C₃H₇ | | | |
| 17 | NC(CH₂)₂ | CH₃O(CH₂)₃ | | | |
| 18 | 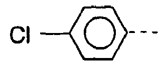 | CH₃ | | | |
| 19 | 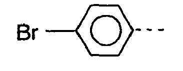 | i-C₃H₇ | Fp. 93-95 | | 55,5 |
| 20 | 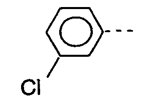 | CH₃ | | | |
| 21 | 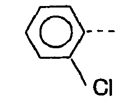 | CH₃ | | | |
| 22 | CH₃ | CH₂=CH-CH₂ | | | |
| 23 | CH₃ | (CH₃)₃C | | | |
| 24 | CH₃ | HC≡C-CH₂ | 9,53; 95 % | 1,4783 | 69,2 |
| 25 | CH₂CH₃ | CH₂=CH-CH₂ | | | |
| 26 | CH₂CH₂CH₂Cl | CH₂=CH-CH₂ | | | |
| 27 | CH(CH₃)₂ | CH₂=CH-CH₂ | 12,19 96,4 % | 1,4735 | 96 |
| 28 | CH₂CH₂CH₂CH₃ | CH₂=CH-CH₂ | | | |
| 29 | CH(CH₃)CH₂CH₃ | CH₂=CH-CH₂ | | | |
| 30 | CH₂CH₃ | HC≡C-CH₂ | | | |
| 31 | CH(CH₃)₂ | HC≡C-CH₂ | 12,27; 97,8 % | 1,4780 | 78,3 |
| 32 | CH₂CH₂CH₂CH₃ | HC≡C-CH₂ | | | |
| 33 | CH₂CH(CH₃)₂ | HC≡C-CH₂ | | | |
| 34 | CH(CH₃) CH₂CH₃ | HC≡C-CH₂ | | | |
| 35 | CH₃ | CH₂CH(CH₃)₂ | | | |
| 36 | CH(CH₃)₂ | CH₂CH₃ | | | |
| 37 | CH (CH₃) | CH₂CH₂CH₂CH₃ | | | |
| 38 | CH(CH₃)₂ | CH₂CH(CH₃)₂ | | | |
| 39 | CH(CH₃)₂ | CH(CH₃) CH₂CH₃ | | | |
| 40 | CH₂-CH=CH-CH₂ | | | | |
| 41 | CH₂-CH(CH₃)-O-CH(CH₃)-CH₂ | | | | |
| 42 | CH₂-CH₂-CH₂-CH₂-CH₂ | | 14,72; 98,3 % | 1,4935 | 90,9 |
| 43 | CH₂-CH₂-CH(CH₃)-CH₂-CH₂ | | 15,32; 98,3 % | 1,4860 | 94,0 |
| 44 | CH₂CH₂CH₃ | HC≡C-CH₂ | | | |
| 45 | Phenyl | CH₃ | 17,75; 94,8 % | 1,5442 | 71,8 |
| 46 | Phenyl | CH₂CH₃ | | | |
| 47 | Cyclohexyl | CH₃ | 17,98; 94,8 % | 1,4960 | 90,8 |
| 48 | Cyclohexyl | CH₂CH₃ | 18,59; 98,6 % | 1,4938 | 63 |

| | | | | | |
|---|---|---|---|---|---|
| 1) (Säule: 25 m Optima 17 GC 9 der Fa. Macherey und Nagel; Druck 14,5 psi; Helium; Säulenfluss 0,6 ml/min; Split, 30 ml/min; Injektor 280°C, Detektor 320°C) 2) Verlustreiche Destillation | | | | | |

### II. Herstellung von Sulfamidsäureamiden

### Beispiel 49: N-Methyl-N-isopropylsulfamidsäureamid

15 g (0,083 Mol) N-Methyl-N-isopropylsulfamidchlorid aus Beispiel 2 wurden innerhalb 5 min bei 0 bis 5°C unter Rühren zu 49 ml (0,654 Mol) 25%igem Ammoniakwasser gegeben und 45 min bei 5 bis 10°C nachgerührt. Nach dem Einengen des Reaktionsgemisches im Vakuum wurde der Rückstand in Methylenchlorid verrührt, von dem unlöslichen Satz abgetrennt, gewaschen und erneut im Vakuum eingeengt. Man erhielt 11,3 g der Titelverbindung vom Sp. 51-53°C. Bezogen auf die im NMR-Spektrum ermittelte Reinheit von 95 % betrug die Ausbeute 84,9 % d. Th.

In analoger Weise wurden die in Tabelle 3 angegebenen Sulfamidsäureamide der Formel III

NH₂-SO₂-NR¹R² (III)

hergestellt (Beispiele 50 bis 63):

**Tabelle 3:**

| Beispiel | R¹ | R² | Fp. [°C] oder n_{D}²³ | Ausbeute [%] |
|---|---|---|---|---|
| 50 | CH₂CH₂CH₃ | CH(CH₃)₂ | 74-76 | 75,8 |
| 51 | HC-C-CH₂ | CH(CH₃)₂ | 1,4850 | 71,6 |
| 52 | CH₂=CH-CH₂ | CH₂CH₂CH₃ | 36-38 | 78,4 |
| 53 | CH₂-CH₂-CH₂-CH₂-CH₂ | | 118-120 | 72,0 |
| 54 | CH₂-CH₂-GH(CH₃)-CH₂-CH₂ | | 126-129 | 68,2 |
| 55 | Cyclohexyl | CH₃ | 117-119 | 77,0 |
| 56 | CH₃CH₂CH₂ | CH₃OCH₂CH₂ | 1, 4694 | 96 |
| 57 | CH₂-CH₂-CH=CH-CH₂ | | 116-118 | 33 |
| 58 | CH₂=C(Cl)CH₂ | CH₂CH₂CH₃ | 40-41 | 33 |
| 59 | CH₂CH(CH₃)CH₂CH₂CH₂ | | 62-64 | 81 |
| 60 | C₂H₅ | CH(CH₃)₂ | 49-51 | 89 |
| 61 | NC-CH₂CH₂ | CH₂CH=CH₂ | 75-77 | 44 |
| 62 | C₆H₅ | CH₃ | 73-76 | 61 |
| 63 | CH(CH₃)₂ | CH₂CH=CH₂ | 48-50 | 77 |

### Beispiel 64

3-(5-(N-Methyl-N-phenyl)-sulfamoylcarboxamid-4-chlor-2-fluorphenyl)-1,2,3,4-tetrahydro-1-methyl-6-trifluormethylpyrimidin-2,4-dion

Zu einer Mischung aus 0,64 g (3,428 mmol) N-Methyl-N-phenyl-sulfamidsäureamid, 0,69 g (6,885 mmol) Triethylamin und einer Spatelspitze p-Dimethylaminopyridin als Katalysator in 40 ml Methylenchlorid gab man bei 0 °C 1,2 g (3,116 mmol) 2-Chlor-4-fluor-5-(3-methyl-2,6-dioxo-4-trifluormethyl-3,6-dihydro-2H-pyrimidin-1-yl)-benzoylchlorid. Man rührte 1 Stunde bei 22°C und extrahierte danach die Reaktionslösung mit 1N Salzsäure. Nach dem Trocknen der organischen Phase über Magnesiumsulfat wurde die organische Phase im Vakuum eingeent. Der erhaltene Rückstand wurde mit Ether verrührt, wobei man 1,3 g (78 % d. Th.) der Titelverbindung mit einem Fp. von 188 - 192 °C erhielt.

Die Herstellung erfolgte in Analogie zu dem auf S. 31 der WO 01/83459 angegebenen Verfahren A.

Analog Beispiel 64 wurden die in Tabelle 4 angegebenen Verbindungen der Formel 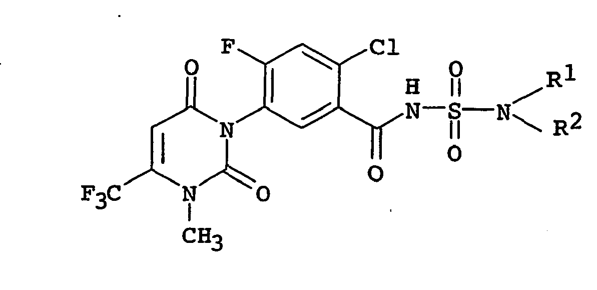 hergestellt (Beispiele 65 bis 71).

**Tabelle 4:**

| Verbindung | R¹ | R² | Fp. [°C] | Ausbeute [%] |
|---|---|---|---|---|
| 65 | CH₂CH=CH₂ | CH₂CH₂CH₃ | 156-158 | 78 |
| 66 | CH₂CH=CH₂ | CH(CH₃)₂ | 138-140 | 66 |
| 67 | CH₂-CH₂-CH₂-CH₂-CH₂ | | 211-213 | 80 |
| 68 | CH₃ | Cyclohexyl | 134-137 | 83 |
| 69 | CH₃ | CH(CH₃)₂ | 91-95 | 45 |
| 70 | CH₂CH=CH₂ | (CH₂)₂CN | 97 | 69 |
| 71 | CH₂CH₂CH₃ | (CH₂)₂OCH₃ | 124-126 | 81 |

## Patentansprüche

1. Verfahren zur Herstellung von Sulfonsäurediamiden durch Umsetzung von Sulfamidsäurehalogeniden primärer oder sekundärer Amine mit Ammoniak, umfassend die folgenden Schritte;
i) Umsetzung eines primären oder sekundären Amins A1 mit wenigstens äquimolaren Mengen SO₃ oder einer SO₃-Quelle in Gegenwart von wenigstens äquimolaren Mengen eines tertiären Amins A2, jeweils bezogen auf das Amin A1;
ii) Umsetzung der in Schritt i) erhaltenen Reaktionsmischung mit wenigstens der stöchiometrisch erforderlichen Menge eines Phosphorhalogenids; und '
iii) Umsetzung des in Schritt ii) erhaltenen Sulfamidsäurehalogenids mit Ammoniak.

2. Verfahren nach Anspruch 1, wobei man wenigstens 2 Mol tertiäres Amin A2 pro Mol Amin A1 einsetzt.

3. Verfahren nach einem der vorhergehenden Ansprüche, wobei man 1,1 Mol SO₃ oder SO₃-Quelle pro Mol Amin A1 einsetzt.

4. Verfahren nach einem der vorhergehenden Ansprüche, wobei man in Schritt i) SO₃ oder ein Addukt eines tertiären Amins A2 an SO₃ als SO₃-Quelle einsetzt.

5. Verfahren nach einem der vorhergehenden Ansprüche, wobei das tertiäre Amin eine Pyridinverbindung ist.

6. Verfahren nach einem der vorhergehenden Ansprüche, wobei das Phosphorhalogenid ausgewählt ist unter Phosphortrichlorid und Phosphoroxychlorid.

7. Verfahren nach Anspruch 6, wobei die Menge an Phosphorhalogenid 1 bis 3 Mol pro Mol Amin A1 beträgt.

8. Verfahren nach einem der Ansprüche 1 bis 5, wobei das Phosphorchlorid ausgewählt ist unter Phosphorpentachlorid.

9. Verfahren nach Anspruch 8, wobei die Menge an Phosphorverbindung 0,5 bis 1 Mol pro Mol Amin A2 beträgt.

10. Verfahren nach einem der vorhergehenden Ansprüche, wobei man Schritt ii) ohne Isolierung des Amidosulfonsäureammoniumsalzes durch Zugabe des Phosphorhalogenids zu der in Schritt i) erhaltenen Reaktionsmischung durchführt.

11. Verfahren nach einem der vorhergehenden Ansprüche, wobei das sekundäre Amin die folgende Formel IB aufweist:
H-NR¹R² (IB)
worin
R¹ und R² unabhängig voneinander für C₁-C₂₀ -Alkyl, C₁-C₂₀-Alkenyl oder C₂-C₂₀-Alkinyl, die unsubstituiert oder durch C₁-C₄-Alkoxy, C₁-C₄-Alkylthio, CN, NO₂, Formyl, C₁-C₄-Alkylcarbonyl, C₁-C₄-Alkoxycarbonyl, C₁-C₄-Alkylaminocarbonyl, C₁-C₄-Dialkylaminocarbonyl, C₁-C₄-Alkylsulfinyl, C₁-C₄-Alkylsulfonyl, C₃-C₁₀-Cycloalkyl, Phenyl, das seinerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen kann, substituiert sein können,
C₁-C₂₀-Halogenalkyl , C₂-C₂₀-Halogenalkenyl, C₂-C₂₀-Halogenalkinyl, C₃-C₁₀-Cycloalkyl, C₅-C₁₀-Cycloalkenyl, Heterocyclyl mit ein bis 3 Heteroatomen, ausgewählt unter O, S und N, Phenyl oder Naphthyl, wobei Heterocyclyl, Phenyl oder Naphthyl, ihrerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, Nitro oder Cyano, aufweisen können,
R¹ und R² auch gemeinsam einen gesättigten oder teilweise ungesättigten 5- bis 8-gliedrigen Stickstoffheterocyclus bilden können, der seinerseits durch C₁-C₄-Alkyl, C₁-C₄-Alkoxy und/oder C₁-C₄-Halogenalkyl, substituiert sein kann, ein oder 2 Carbonylgruppen, Thiocarbonylgruppen und/oder ein oder zwei weitere Heteroatome, ausgewählt unter O, S und N, als Ringglieder aufweisen kann.

12. Verfahren nach einem der vorhergehenden Ansprüche zur Herstellung von Verbindungen der allgemeinen Formel IV, 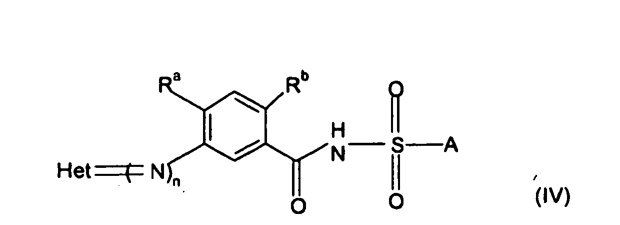 worin n für 0 oder 1 steht, Het einen gegebenenfalls substituierten 5- oder 6-gliedrigen Heterocyclus bedeutet, der 1, 2 oder 3 Stickstoffatome, gegebenenfalls ein weiteres Heteroatom ausgewählt unter Sauerstoff und Schwefel, und gegebenenfalls ein oder zwei Carbonyl- oder Thiocarbonylgruppen als Ringglieder aufweisen kann, R^{a} für Wasserstoff, Fluor oder Chlor, R^{b} für Chlor oder Cyano und A für einen von einem primären oder sekundären Amin A1 abgeleiteten Rest steht, umfassend die folgenden Schritte:
i) Umsetzung eines primären oder sekundären Amins A1 mit wenigstens äquimolaren Mengen SO₃ oder einer SO₃-Quelle in Gegenwart von wenigstens äquimolaren Mengen eines tertiären Amins A2, jeweils bezogen auf das Amin A1, wobei man ein Amidosulfonsäureammoniumsalz erhält;
ii) Umsetzung des Amidosulfonsäureammoniumsalz mit wenigstens der stöchiometrisch erforderlichen Menge eines Phosphorhalogenids, wobei man ein Sulfamidsäurehalogenid des Amins A1 erhält;
iii)Umsetzung des in Schritt ii) erhaltenen Sulfamidsäurehalogenids mit Ammoniak, wobei man ein Sulfamidsäureamid erhält; und
iv) Umsetzung des Reaktionsprodukts aus Schritt iii) mit einer Verbindung der allgemeinen Formel V 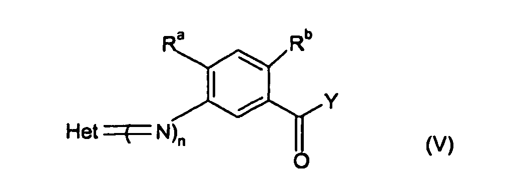
worin Het, n, R^{a} und R^{b} die für Formel IV angegebenen Bedeutungen aufweisen und Y für OH, Alkoxy oder ein Halogenatom steht.

13. Verfahren nach Anspruch 12, worin n = 0 ist und Het für einen Rest der Formel Steht.

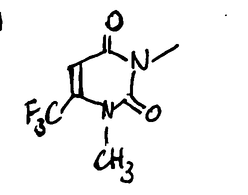

## Claims

1. A process for preparing sulfuric diamides by reacting sulfamoyl halides of primary or secondary amines with ammonia, comprising the following steps:
i) reacting a primary or secondary amine A1 with at least equimolar amounts of SO₃ or an SO₃ source in the presence of at least equimolar amounts of a tertiary amine A2, based in each case on the amine A1;
ii) reacting the reaction mixture obtained in step i) with at least the amount of a phosphorus halide required by the stoichiometry; and
iii) reacting the sulfamoyl halide obtained in step ii) with ammonia.

2. The process according to claim 1, wherein at least 2 mol of tertiary amine A2 are used per mole of amine A1.

3. The process according to either of the preceding claims, wherein 1.1 mol of SO₃ or SO₃ source are used per mole of amine A1.

4. The process according to any of the preceding claims, wherein the SO₃ source used in step i) is SO₃ or an adduct of a tertiary amine A2 with SO₃.

5. The process according to any of the preceding claims, wherein the tertiary amine is a pyridine compound.

6. The process according to any of the preceding claims, wherein the phosphorus halide is selected from phosphorus trichloride and phosphorus oxychloride.

7. The process according to claim 6, wherein the amount of phosphorus halide is from 1 to 3 mol per mole of amine A1.

8. The process according to any of claims 1 to 5, wherein the phosphorus chloride is phosphorus pentachloride.

9. The process according to claim 8, wherein the amount of phosphorus compound is from 0.5 to 1 mol per mole of amine A2.

10. The process according to any of the preceding claims, wherein step ii) is carried out without isolating the ammonium sulfamate by adding the phosphorus halide to the reaction mixture obtained in step i).

11. The process according to any of the preceding claims, wherein the secondary amine has the following formula IB:
H-NR¹R² (IB)
where
R¹ and R² are each independently C₁-C₂₀-alkyl, C₂-C₂₀-alkenyl or C₂-C₂₀-alkynyl, each of which may be unsubstituted or substituted by C₁-C₄-alkoxy, C₁-C₄-alkylthio, CN, NO₂, formyl, C₁-C₄-alkylcarbonyl, C₁-C₄-alkoxycarbonyl, C₁-C₄-alkylaminocarbonyl, C₁-C₄-dialkylaminocarbonyl, C₁-C₄-alkylsulfinyl, C₁-C₄-alkylsulfonyl, C₃-C₁₀-cycloalkyl, phenyl which may itself have 1, 2, 3 or 4 substituents selected from halogen, C₁-C₄-alkyl, C₁-C₄-alkoxy, C₁-C₄-fluoroalkyl, C₁-C₄-alkyloxycarbonyl, trifluoromethylsulfonyl, formyl, nitro or cyano,
C₁-C₂₀-haloalkyl, C₂-C₂₀-haloalkenyl, C₂-C₂₀-haloalkynyl, C₃-C₁₀-cycloalkyl, C₅-C₁₀-cycloalkenyl, heterocyclyl having from one to 3 heteroatoms selected from O, S and N, phenyl or naphthyl, where heterocyclyl, phenyl or naphthyl may themselves have 1, 2, 3 or 4 substituents selected from halogen, C₁-C₄-alkyl, C₁-C₄-alkoxy, C₁-C₄-fluoroalkyl, C₁-C₄-alkyloxycarbonyl, trifluoromethylsulfonyl, formyl, nitro or cyano,
R¹ and R² together may also form a saturated or partially unsaturated 5- to 8-membered nitrogen heterocycle which may itself be substituted by C₁-C₄-alkyl, C₁-C₄-alkoxy and/or C₁-C₄-haloalkyl, and have one or 2 carbonyl groups or thiocarbonyl groups and/or one or two further heteroatoms selected from O, S and N as ring members.

12. The process according to any of the preceding claims for preparing compounds of the general formula IV 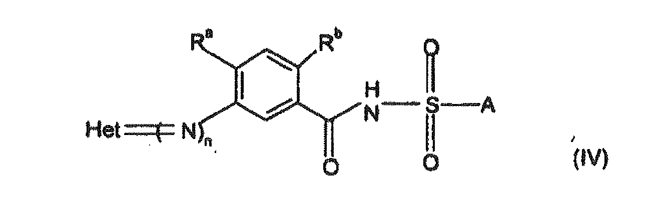 where n is 0 or 1, Het is an optionally substituted 5- or 6-membered heterocycle which may have 1, 2 or 3 nitrogen atoms, optionally one further heteroatom selected from oxygen and sulfur, and optionally one or two carbonyl or thiocarbonyl groups as ring members, R^{a} is hydrogen, fluorine or chlorine, R^{b} is chlorine or cyano, and A is a radical derived from a primary or secondary amine A1, comprising the following steps:
i) reacting a primary or secondary amine A1 with at least equimolar amounts of SO₃ or an SO₃ source in the presence of at least equimolar amounts of a tertiary amine A2, based in each case on the amine A1, to obtain an ammonium sulfamate;
ii) reacting the ammonium sulfamate with at least the amount of a phosphorus halide required by the stoichiometry, to obtain a sulfamoyl halide of the amine A1;
iii) reacting the sulfamoyl halide obtained in step ii) with ammonia to obtain a sulfamoyl amide; and
iv) reacting the reaction product from step iii) with a compound of the general formula V 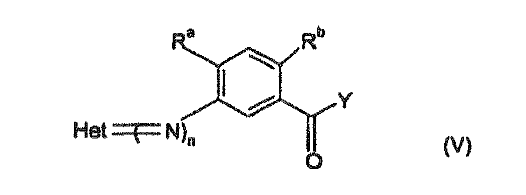
where Het, n, R^{a} and R^{b} are each as defined for formula IV and Y is OH, alkoxy or a halogen atom.

13. The process according to claim 12, where n = 0 and Het is a radical of the formula

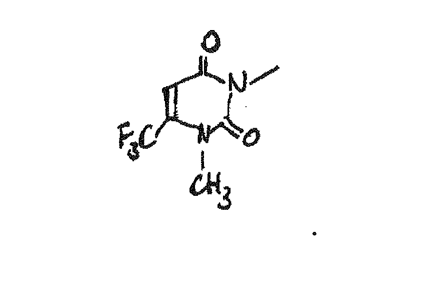

## Revendications

1. Procédé de fabrication de diamides de l'acide sulfonique par réaction d'halogénures de l'acide sulfamidique d'amines primaires ou secondaires avec de l'ammoniac, comprenant les étapes suivantes :
i) réaction d'une amine primaire ou secondaire A1 avec au moins des quantités équimolaires de SO₃ ou d'une source de SO₃ en présence d'au moins des quantités équimolaires d'une amine tertiaire A2, à chaque fois par rapport à l'amine A1 ;
ii) réaction du mélange réactionnel obtenu à l'étape i) avec au moins la quantité stoechiométriquement nécessaire d'un halogénure de phosphore ; et
iii) réaction de l'halogénure de l'acide sulfamidique obtenu à l'étape ii) avec de l'ammoniac.

2. Procédé selon la revendication 1, dans lequel au moins 2 moles d'amine tertiaire A2 sont utilisées par mole d'amine A1.

3. Procédé selon l'une quelconque des revendications précédentes, dans lequel 1,1 moles de SO₃ ou de source de SO₃ sont utilisées par mole d'amine A1.

4. Procédé selon l'une quelconque des revendications précédentes, dans lequel du SO₃ ou un produit d'addition d'une amine tertiaire A2 sur SO₃ est utilisé en tant que source de SO₃ à l'étape i).

5. Procédé selon l'une quelconque des revendications précédentes, dans lequel l'amine tertiaire est un composé de pyridine.

6. Procédé selon l'une quelconque des revendications précédentes, dans lequel l'halogénure de phosphore est choisi parmi le trichlorure de phosphore et l'oxychlorure de phosphore.

7. Procédé selon la revendication 6, dans lequel la quantité d'halogénure de phosphore est de 1 à 3 moles par mole d'amine A1.

8. Procédé selon l'une quelconque des revendications 1 à 5, dans lequel le chlorure de phosphore est choisi parmi le pentachlorure de phosphore.

9. Procédé selon la revendication 8, dans lequel la quantité de composé de phosphore est de 0,5 à 1 mole par mole d'amine A2.

10. Procédé selon l'une quelconque des revendications précédentes, dans lequel l'étape ii) est réalisée sans isolement du sel d'ammonium de l'acide amidosulfonique par ajout de l'halogénure de phosphore au mélange réactionnel obtenu à l'étape i).

11. Procédé selon l'une quelconque des revendications précédentes, dans lequel l'amine secondaire présente la formule IB suivante
H-NR¹R² (IB)
dans laquelle
R¹ et R² représentent indépendamment l'un de l'autre alkyle en C₁-C₂₀, alcényle en C₂-C₂₀ ou alcynyle en C₂-C₂₀, qui peuvent être non substitués ou substitués par alcoxy en C₁-C₄, alkylthio en C₁-C₄, CN, NO₂, formyle, alkylcarbonyle en C₁-C₄, alcoxycarbonyle en C₁-C₄, alkylaminocarbonyle en C₁-C₄, dialkylaminocarbonyle en C₁-C₄, alkylsulfinyle en C₁-C₄, alkylsulfonyle en C₁-C₄, cycloalkyle en C₃-C₁₀, phényle, qui peut comporter pour sa part 1, 2, 3 ou 4 substituants, choisis parmi halogène, alkyle en C₁-C₄, alcoxy en C₁-C₄, fluoroalkyle en C₁-C₄, alkyloxycarbonyle en C₁-C₄, trifluorométhylsulfonyle, formyle, nitro ou cyano, halogénoalkyle en C₁-C₂₀, halogénoalcényle en C₂-C₂₀, halogénoalcynyle en C₂-C₂₀ , cycloalkyle en C₃-C₁₀, cycloalcényle en C₅-C₁₀, hétérocyclyle avec un à 3 hétéroatomes, choisis parmi O, S et N, phényle ou naphtyle, hétérocyclyle, phényle ou naphtyle pouvant comporter pour leur part 1, 2, 3 ou 4 substituants choisis parmi halogène, alkyle en C₁-C₄, alcoxy en C₁-C₄, fluoroalkyle en C₁-C₄, alkyloxycarbonyle en C₁-C₄, trifluorométhylsulfonyle, formyle, nitro ou cyano,
R¹ et R² pouvant également former ensemble un hétérocycle azoté à 5 à 8 éléments saturé ou partiellement insaturé, qui être substitué pour sa part par alkyle en C₁-C₄, alcoxy en C₁-C₄ et/ou halogénoalkyle en C₁-C₄, qui peut comporter un ou 2 groupes carbonyle, groupes thiocarbonyle et/ou un ou deux hétéroatomes supplémentaires, choisis parmi O, S et N, en tant qu'élément de cycle.

12. Procédé selon l'une quelconque des revendications précédentes, pour la fabrication de composés de formule générale IV, 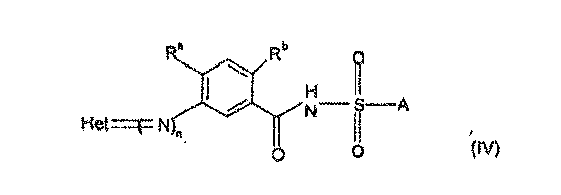 dans laquelle n représente 0 ou 1, Het représente un hétérocycle à 5 ou 6 éléments éventuellement substitué, qui peut comporter 1, 2 ou 3 atomes d'azote, éventuellement un hétéroatome supplémentaire choisi parmi l'oxygène et le soufre, et éventuellement un ou deux groupes carbonyle ou thiocarbonyle en tant qu'élément de cycle, R^{a} représente hydrogène, fluor ou chlore, R^{b} représente chlore ou cyano, et A représente un radical dérivé d'une amine primaire ou secondaire A1, comprenant les étapes suivantes :
i) réaction d'une amine primaire ou secondaire A1 avec au moins des quantités équimolaires de SO₃ ou d'une source de SO₃ en présence d'au moins des quantités équimolaires d'une amine tertiaire A2, à chaque fois par rapport à l'amine A1, un sel d'ammonium de l'acide amidosulfonique étant obtenu ;
ii) réaction du sel d'ammonium de l'acide amidosulfonique avec au moins la quantité stoechiométriquement nécessaire d'un halogénure de phosphore, un halogénure de l'acide sulfamidique de l'amine A1 étant obtenu ;
iii) réaction de l'halogénure de l'acide sulfamidique obtenu à l'étape ii) avec de l'ammoniac, un amide de l'acide sulfamidique étant obtenu ; et
iv) réaction du produit de la réaction de l'étape iii) avec un composé de formule générale V 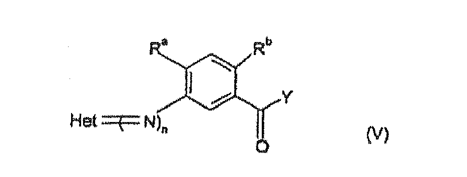
dans laquelle Het, n, R^{a} et R^{b} présentent les significations données pour la formule IV, et Y représente OH, alcoxy ou un atome d'halogène.

13. Procédé selon la revendication 12, dans lequel n = 0 et Het représente un radical de formule
